(19) Europäisches Patentamt / European Patent Office / Office européen des brevets

(11) **EP 4 252 747 A1**

(12) **EUROPEAN PATENT APPLICATION**
published in accordance with Art. 153(4) EPC

(43) Date of publication:
04.10.2023 Bulletin 2023/40

(21) Application number: 21898562.0

(22) Date of filing: 23.11.2021

(51) International Patent Classification (IPC):
*A61K 9/51* (2006.01)     *A61K 31/437* (2006.01)
*A61K 31/4745* (2006.01)     *A61K 45/06* (2006.01)
*A61P 35/00* (2006.01)

(52) Cooperative Patent Classification (CPC):
A61K 9/51; A61K 31/437; A61K 31/4745;
A61K 45/06; A61P 35/00

(86) International application number:
PCT/KR2021/017296

(87) International publication number:
WO 2022/114736 (02.06.2022 Gazette 2022/22)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 27.11.2020 KR 20200163156

(71) Applicant: SNBioscience Inc.
Seongnam-si, Gyeonggi-do, 13449 (KR)

(72) Inventor: PARK, Young Hwan
Seoul 06374 (KR)

(74) Representative: J A Kemp LLP
80 Turnmill Street
London EC1M 5QU (GB)

(54) **PHARMACEUTICAL COMPOSITION COMPRISING INSOLUBLE CAMPTOTHECIN COMPOUND-CONTAINING NANOPARTICLE FOR TREATMENT OF CANCER AND COMBINATION THERAPY THEREOF**

(57) The present disclosure relates to a use of poorly soluble camptothecin-based compound-comprising nanoparticles for cancer treatment, and a combination therapy using same together with an antitumor agent. A pharmaceutical composition comprising the nanoparticles of the present invention exhibits a synergistic cancer treatment effect when used in combination with an antitumor agent and as such, can be easily used as a cancer therapeutic agent and a combination therapy formulation.

FIG.2

EP 4 252 747 A1

## Description

Technical Field

This patent application claims priority to and the benefit of Korean Patent Application No. 110-2020-0163156 filed with the Korean Intellectual Property Office on November 27, 2020, the disclosure of which is incorporated herein by reference.

The present disclosure relates to a pharmaceutical composition comprising poorly soluble camptothecin compound-comprising nanoparticles for treatment of cancer and a combination therapy thereof.

Background Art

Cancer treatments include surgery, radiotherapy, and drug treatments (such as chemotherapy or hormone therapy) . For malignant tumors, when primary treatment surgery is not possible, chemotherapy is selected (alone or in combination). Cytotoxic anticancer agents used for chemotherapy of malignant tumors worldwide include camptothecin-based anticancer drugs such as the DNA topoisomerase inhibitor irinotecan, gemcitabine, which is a type of antimetabolites, and paclitaxel, which is a microtubule inhibitor.

Irinotecan, known as CPT-11, is a semi-synthetic and water-soluble analogue of the natural alkaloid camptothecin, and blocks topoisomerase's DNA unwinding to prevent DNA replication, thereby inhibiting cell proliferation. Irinotecan is currently formulated as an aqueous solution and sold under the brand name Camptosar™ (irinotecan hydrochloride injection). Irinotecan is a prodrug-type anticancer drug, and is converted by carboxylesterase 2 (CES2) into SN-38, an active metabolite, in the body. The active form SN-38 has anticancer activity 100 to 1,000 times higher than that of irinotecan, but is unstable at body pH and difficult to develop into a formulation due to its extremely poor solubility. In addition, the conversion rate of irinotecan to SN-38 in the body is as very low as 2-8%, with a large variation (more than 4 times) among patients. Thus, as a cytotoxic anticancer drug that must be calculated for precise dosage (mg/m2) for each patient, irinotecan has the disadvantage that its exact effects or side effects cannot be predicted. The active form SN-38 is a very poorly soluble material and is not dissolved by a general solubilization method, so a lot of solubilization research and development is currently in progress. Carboxylesterase 2 (CES2), which converts irinotecan into the active SN-38, shows a large deviation in expression rate from one tumor tissue to another. CES2 is most expressed in the small intestine and colon, which are the main indications for irinotecan, but CES2 is hardly expressed in pancreatic cancer, gallbladder cancer, breast cancer, lung cancer, kidney cancer, prostate cancer, etc. Therefore, if the active form SN-38 can be directly administered, it is expected that the anticancer effect can be exerted even on the tumor tissue, which hardly expresses CES2RK.

Meanwhile, gemcitabine is a useful anticancer agent and is currently used as the first-line standard treatment for pancreatic cancer. However, the tumor suppressor efficacy is low, and the effect of extending the survival period of cancer patients is also low. In addition, monotherapy with taxane-based anticancer agents such as paclitaxel or nab-paclitaxel (albumin nanoparticulate paclitaxel) has different sensitivities depending on the tumor, so there is a limitation in that the anticancer effect is not constant for each patient. Therefore, in order to overcome the clinical limitations of each cytotoxic anticancer drug monotherapy, research is being actively conducted to augment drug efficacy and increase safety by improving drug delivery technology or combining anticancer drugs different in mechanism of action.

Throughout the entire specification, many patent documents are referenced and their citations are represented. The disclosures of cited papers and patent documents are entirely incorporated by reference into the present specification, and the level of the technical field within which the present disclosure falls and details of the present disclosure are explained more clearly.

Disclosure of Invention

Technical Problem

In general, it is known that the low antitumor effect of chemotherapy is not only because the drug's blood residence time is short and the selective drug delivery efficiency to the tumor tissue is low, but also because the sensitivity to drugs varies greatly for each tumor due to the characteristics of tumors with high mutation rates. Therefore, the present inventors made intensive and thorough research into the development of a combination therapy adapted for employing antitumor agents with different mechanisms of action in combination and effectively delivering the antitumor agents of high antitumor activity to increase the sensitivity against tumors, with the consequent achievement of maximum antitumor efficacy as well as improved safety. As a result, it was found that an excellent antitumor effect was obtained when an antitumor agent and a nanoparticle formulation comprising hydrophobic camptothecin and hydrophilic camptothecin were used in combination, leading to the present disclosure.

Accordingly, an aspect of the present disclosure is to provide a pharmaceutical composition for the treatment of cancer, which is to be administered in combination with an antitumor agent.

Another aspect of the present disclosure is to provide a pharmaceutical combination preparation that includes: particles comprising a hydrophobic camptothecin-based compound, a hydrophilic camptothecin-based compound, and an amphiphilic block copolymer composed of a hydrophobic block and a hydrophilic block; and an antitumor agent as an active ingredient, and is administered simultaneously, separately or sequentially for cancer treatment.

Solution to Problem

According to one aspect thereof, the present disclosure provides a pharmaceutical composition for the treatment of cancer, which is administered in combination with an antitumor agent.

The pharmaceutical composition of the present disclosure includes, as an active ingredient, particles comprising a hydrophobic camptothecin-based compound, a hydrophilic camptothecin-based compound, and an amphiphilic block copolymer composed of a hydrophobic block and a hydrophilic block.

Herein, camptothecin is a topoisomerase inhibitor found in bark and stalk of the Camptotheca tree (Happy tree) and exhibits a very excellent anticancer effect in the preclinical stage, but cannot be used due to low solubility thereof. Therefore, many researchers have developed camptothecin analogues for improving the solubility of camptothecin. Currently, three types of camptothecin derivatives, irinotecan, topotecan, and belotecan, have been approved, and used in chemotherapy for cancer.

As used herein, the term "hydrophobicity" refers to the exclusion from water molecules and agglomeration, as a tendency shown in non-polar materials. When a hydrophobic material is present in a hydrophilic liquid, the hydrophobic material agglomerates while increasing hydrophobic bonds as if the hydrophobic material is afraid of water.

As used herein, the term "hydrophilicity" refers to the property of being dissolved in a polar solvent, such as water, with strong affinity therewith, as a tendency shown in polar materials. For example, a hydrophilic polymer compound, or a micelle colloid surface of a surfactant has strong hydrophilicity.

In an embodiment of the present disclosure, the hydrophobic camptothecin-based compound is at least one selected from the group consisting of 7-ethyl-10-hydroxycamptothecin (SN-38), camptothecin, 10-hydroxycamptothecin, and a pharmaceutical acceptable salt thereof, but is not limited thereto.

In another embodiment of the present disclosure, the hydrophilic camptothecin-based compound is selected from irinotecan, topotecan, belotecan, exatecan, lurtotecan, sinotecan, rubitecan, 9-nitrocamptothecin, 9-aminocamptothecin, gimatecan, karenitecin, silatecan, diflomotecan, elomotecan, a pharmaceutically acceptable salt thereof, a glucuronide metabolite thereof, and a glucuronide metabolite of the hydrophobic camptothecin-based compound, but is not limited thereto. The above-mentioned hydrophilic camptothecin-based compound may include a type of compound generally classified as a hydrophobic drug. The "hydrophilicity" should be understood to mean relative hydrophilicity compared to the above-described types of hydrophobic camptothecin-based compounds among components constituting the particles of the present disclosure. That is, the hydrophilic camptothecin-based compound refers to a relatively hydrophilic camptothecin-based compound compared to the hydrophobic camptothecin-based compound included in the particles of the present disclosure.

According to a specific embodiment of the present disclosure, the hydrophobic camptothecin-based compound, which constitutes the particle of the present disclosure, may be camptothecin, SN-38, or a mixture thereof, and the hydrophilic camptothecin-based compound, which constitutes the particle of the present disclosure, may be irinotecan hydrochloride, topotecan hydrochloride, and a glucuronide analogue of SN-38.

As used herein, the term "copolymer" refers to a polymer prepared from two or more different types of monomers. For example, the reaction of styrene acrylonitrile in a reaction container results in a copolymer having both of the two monomers. The term "block copolymer" refers to a copolymer having a form in which a block of one type of monomers is linked to a block of another type of monomers. A case in which a block of material A is followed by a block of material B is expressed by -[-AB-]-. A chain is called AB type if the chain is composed of only one strand of each monomer, ABA type if A blocks are present at both ends of B block in the center, and ABC type if three different types of blocks are present in a main chain. A block copolymer is mainly formed by ionic polymerization. Unlike other copolymers, this block copolymer has many physical properties of a homopolymer formed from two types of monomers.

In an embodiment of the present disclosure, the amphiphilic block copolymer constituting the particle of the present disclosure is composed of A-B blocks or A-B-A blocks. Here, A is a hydrophilic polymer, which is monomethoxy polyethylene glycol, dimethoxy polyethylene glycol, polyethylene glycol, polypropylene glycol, monomethoxy polypropylene glycol, polyethylene oxide, polyacrylic acid, or a polymer thereof, but is not limited thereto.

In addition, B is a hydrophobic polymer, which is polylactic acid, poly-L-lactide, poly-D-lactide, poly-D, L-lactide, poly(lactide-co-glycolide), polyglycolic acid, polyglycolide, a polylactic acid-glycolic acid copolymer, polymandelic acid, polycaprolactone, polydioxan-2-one, polyglutamic acid, polyaspartic acid, polyornithine, polyorthoester, a derivative thereof, or a copolymer of one or more compounds selected therefrom, but is not limited thereto. It would be obvious to a person skilled in the art that any compound that can constitute an amphiphilic block copolymer usable in the art can be used without limitation.

In a specific embodiment of the present disclosure, the amphiphilic block copolymer is mPEG-PDLLA (mPEG-Poly(D,L) Lactic Acid); PEG-PCL [poly(ethylene glycol)-b-poly(carprolactone)]; PEG-PLA [poly(ethylene glycol)-b-poly(lactic acid)]; mPEG-PGA [monomethoxy poly(ethylene glycol)-b-poly(glycolic acid)]; mPEG-PLGA [monomethoxy poly(ethylene glycol)-b-poly(lactide-co-glycolide)]; PEG-PBLA [poly(ethylene glycol)-b-poly($\beta$-benzyl-L-aspartic acid)]; PEG-p(Glu) [poly(ethylene glycol)-b-poly(glutamic acid)]; PEG-p(Asp) [poly(ethylene glycol)-b-poly(aspartic acid)]; and/or PEG-PLA-PEG [poly(ethylene glycol)-b-poly(lactic acid)-b-poly(ethylene glycol)].

According to the most specific embodiment of the present disclosure, the properties of the particles and manufacturing method therefor are described in detail in Korean Patent No. 10-2094543 issued to present applicant, the disclosure of which is incorporated herein by reference.

Particles, prepared according to the patent or the following Examples, comprising SN-38 as a hydrophobic camptothecin-based compound and irinotecan hydrochloride as a hydrophilic camptothecin-based compound, and mPEG-PDLLA as an amphiphilic block copolymer was named SNB-101.

In another embodiment of the present disclosure, the weight ratio of the hydrophobic camptothecin-based compound and the hydrophilic camptothecin-based compound, which constitute the particle of the present disclosure, is 1-10:1-10, 1-10:1-5, 1-10:1-3, 1-10:1, 1-5:1-10, 1-3:1-10, or 1:1-10, specifically 1-5:1-5, 1-5:1-3, 1-5:1, 1-3:1-5, or 1:1-5, and more specifically 1-3:1-3, 1-3:1, or 1:1-3, but is not limited thereto.

In a specific embodiment of the present disclosure, the weight ratio of the hydrophobic camptothecin-based compound to the hydrophilic camptothecin-based compound is 1:1-10, 1:1-5, 1:1-3, or 1:1-2 may be, and most specifically, may be 1:1.59, but is not limited thereto.

In an embodiment of the present disclosure, the weight ratio of (a) the sum of the hydrophobic camptothecin-based compound and the hydrophilic camptothecin-based compound and (b) the amphiphilic block copolymer is 1:0.1-200, 1:0.5-200, 1:1-200, 1:2-200, 1:5-200, 1:10-200, 1:50-200, 1:100-200, 1:150-200, 1:0.1-100, 1:0.5-100, 1:1-100, 1:2-100, 1:5-100, 1:10-100, 1:20-100, 1:50-100, 1:0.1-50, 1:0.5-50, 1:1-50, 1:5-50, 1:10-50, 1:20-50, 1:0.1-20, 1:0.5-20, 1:1-20, 1:5-20, 1:10-20, 1:0.1-10, 1:0.5-10, or 1:1-10, but with no limitations thereto.

As used herein, the term "to", "-", or "~" between two numerical values means a section between the numerical values including numerical values described before and after the term.

In an embodiment of the present disclosure, the particles of the present disclosure are prepared by the following method. First, hydrophobic camptothecin (e.g., SN-38) is put, together with hydrophilic camptothecin (e.g., irinotecan hydrochloride), in an organic solvent and completely dissolved by stirring, and an amphiphilic block copolymer (e.g., mPEG-PDLLA) previously dissolved in an organic solvent was added thereto while stirring. The mixture is dried with a rotary evaporation vacuum dryer or vacuum dryer. An aqueous solvent (e.g., distilled water, PBS) is added to the residue, followed by ultrasonication in an ultrasonic cleaner to prepare the particles of the present disclosure.

In an embodiment of the present disclosure, the organic solvent is an alcohol of C1 to C5 (methanol, ethanol, propanol, butanol, n-butanol, iso-propanol, 1-pentanol, 2-butoxyethanol, isobutylalcohol, and so on) alkyl acetate, acetone, acetonitrile, chloroform, benzene, toluene, xylene, acetone, fluoroalkane, pentane, hexane, 2,2,4-trimethylpentane, decane, cyclohexane, cyclopentane, diisobutylene, 1-pentene, 1-chlorobutane, 1-chloropentane, diisopropyl ether, 2-chloropropane, 1-chloropropane, chlorobenzene, benzene, diethyl ether, diethyl sulfide, dichloromethane, 1,2-dichloroethane, aniline, diethylamine, ether, carbon tetrachloride, tetrahydrofuran (THF) or a mixture thereof, but with no limitations thereto.

The pharmaceutical composition of the present disclosure includes an antitumor agent.

The antitumor agent includes at least one selected from the group consisting of a taxane-based anticancer agent, an albumin-bound taxane-based anticancer agent, a vascular endothelial growth factor (VEGF) inhibitor, and gemcitabine.

In an embodiment of the present disclosure, the taxane-based anticancer agent may be at least one selected from the group consisting of paclitaxel, docetaxel, larotaxel, cabazitaxel, and pharmaceutically acceptable salts thereof, but is not limited thereto.

In an embodiment of the present disclosure, the albumin-bound taxane-based anticancer agent is at least one selected from the group consisting of albumin-binding paclitaxel (albumin nanoparticles paclitaxel or nab-paclitaxel), and albumin-bound docetaxel (albumin nanoparticles docetaxel or nab-docetaxel), but with no limitations thereto.

In an embodiment of the present disclosure, the vascular endothelial growth factor (VEGF) inhibitor may be at least one selected from the group consisting of bevacizumab, ranibizumab, and aflibercept, but with no limitations thereto.

In a specific embodiment of the present disclosure, the hydrophobic camptothecin-based compound is SN-38, the hydrophilic camptothecin-based compound is irinotecan hydrochloride, and the amphiphilic block copolymer is mPEG-PDLLA (mPEG-Poly(D, L) lactic acid).

The particles of the present disclosure prepared according to the above specific embodiment were designated SNB-101 by the present inventors.

The stable nanoparticle formulation (SNB-101) of SN-38 and irinotecan hydrochloride is a formulation obtained by solubilizing and formulating SN-38, which has been difficult to use due to its extremely poorly solubility in spite of the excellent anticancer effect, allows SN-38 to be directly administered for pancreatic cancer, ovarian cancer, lung cancer, breast cancer, etc., which lack carboxylesterase, with expectancy for new indications.

As for the particles, used an active ingredient in the pharmaceutical composition of the present disclosure, according to a specific embodiment of the present disclosure, SN-38 is preferably contained in an amount of 0.1 to 1 mg/ml and more preferably in an amount of 0.2 to 1.0 mg/ml in the particle composition. In addition, irinotecan hydrochloride is preferably contained in an amount of 0.1-2 mg/ml and more preferably in an amount of 0.2-1.6 mg/ml in the particle composition

In one embodiment of the present disclosure, the particle has a double structure. The double structure may include an inner structure accounted for by the hydrophilic camptothecin-based compound and the hydrophobic camptothecin-based compound; and an outer structure formed by the amphiphilic block copolymer.

In a fabricating process for the particles having the double structure of the present disclosure, first, the hydrophilic camptothecin-based compound and the hydrophobic camptothecin-based compound are mixed and dissolved in an organic solvent, and a solution of an amphiphilic polymer (amphiphilic block copolymer) in an organic solvent is added to the mixture while stirring, and then dried. When the residues thus obtained is emulsified in an aqueous solvent, such as by sonication, water-dispersible nanoparticles are formed.

The amphiphilic block copolymer in which a hydrophilic block and a hydrophobic block are combined at a particular ratio is known to form a micelle-like water-dispersible particle through self-assembly in an aqueous solution. It is assumed that the water-dispersible particles of the present disclosure include the hydrophilic camptothecin-based compound and hydrophobic camptothecin-based compound in the inner portion thereof and the amphiphilic block copolymer responsible for the outer shell thereof. More specifically, in the structure of the water-dispersible particles of the present disclosure, the hydrophobic block of the amphiphilic block copolymer faces the inner structure formed by the relatively hydrophobic camptothecin-based compounds while the hydrophilic block is directed toward the external aqueous solvent, thus forming a shell.

In an embodiment of the present disclosure, the water-dispersible particles of the double structure of the present disclosure are characterized in that they spontaneously form particles when dispersed in an aqueous solution.

In another embodiment of the present disclosure, the particles have an average diameter of 2-200 nm. In the case where the particles manufactured according to the present disclosure have a particle size of 200 nm or less, the non-selective removal of a reticuloendothelial (RES) system in the body can be avoided, and thus it is preferable to manufacture particles having a uniform particle size of 200 nm or less.

In an embodiment of the present disclosure, the water-dispersible particles of the present disclosure may be mixed with a cryoprotective agent such as trehalose or mannitol before lyophilization.

With the average diameter (particle size) of 200 nm or less, the particles according to the present disclosure can maximize the delivery of the drugs to tumor tissues by taking advantage of the characteristic enhanced permeation and retention (EPR) of tumor tissues. As for EPR, the normal integrity of the vascular system (especially capillaries) is impaired, so that particles such as nano-sized particle carriers (nanoparticles, liposomes, micelles) leak from the capillary and is deposited on the tumor site.

Therefore, due to the characteristics of the particles (SNB-101) of the present disclosure, SN-38 and irinotecan contained in SNB-101 can be present in the blood for a long time, thereby promoting the accumulation of the drug into the tumor tissue. It is thus expected that the maximum tolerated dose (MTD) of the drug can be increased by increasing the drug exposure to cancer cells in the tumor and reducing the drug exposure to normal tissues, thereby improving the safety.

In an embodiment of the present disclosure, the pharmaceutical composition according to one aspect of the present disclosure is for the treatment of cancer. The cancer may be selected from the group consisting of gastric cancer, ovarian cancer, uterine cancer, cervical cancer, small cell lung cancer, non-small cell lung cancer, pancreatic cancer, breast cancer, esophageal cancer, retinoblastoma, oral cancer, salivary gland cancer, laryngeal cancer, pharyngeal cancer, rectal cancer, colon cancer, colorectal cancer, kidney cancer, prostate cancer, melanoma, liver cancer, gallbladder and other biliary tract cancer, thyroid cancer, bladder cancer, brain and central nervous system cancer, bone tumor, skin cancer, non-Hodgkin's lymphoma, non-Hodgkin's lymphoma, but with no limitations thereto. The brain cancer may be glioma, meningioma, schwannoma, a pituitary tumor, a metastatic brain tumor, or a base cranial tumor. The glioma includes astrocytoma, oligodendroglioma, ependymoma, or a mixture thereof.

In a specific embodiment of the present disclosure, the cancer that is the target disease of the pharmaceutical composition is pancreatic cancer, stomach cancer, breast cancer, lung cancer, colorectal cancer, or a combination thereof.

When the particles of the present disclosure or the composition comprising the same is prepared into a pharmaceutical composition, the pharmaceutical composition of the present disclosure may contain a pharmaceutically acceptable carrier. The pharmaceutically acceptable carrier is normally used at the time of formulation, and examples thereof may include, but are not limited to, lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methylcellulose, methyl hydroxybenzoate, propyl hydroxybenzoate, talc, magnesium stearate, and mineral oil. The pharmaceutical composition of the present disclosure may further comprise a lubricant, a wetting agent, a sweetening agent, a flavoring agent, an emulsifier, a suspending agent, a preservative, and the like, in addition to the above ingredients. Suitable pharmaceutically acceptable carriers and agents are described in detail in Remington's Pharmaceutical Sciences (19th ed., 1995).

In a specific embodiment of the present disclosure, the pharmaceutical composition of the present disclosure may further comprise sucrose, mannitol, sorbitol, glycerin, trehalose, a polyethylene glycol-based excipient, and a cyclodextrin-based excipient (alpha-, beta-, and gamma-cyclodextrin, hydroxy cyclodextrin, or a cyclodextrin derivative). The excipient is added to the particles, which correspond to an active ingredient of the present pharmaceutical composition, serves as a cryoprotectant or an osmoregulator, and is formulated by freeze-drying, solvent evaporation, or the like.

The pharmaceutical composition of the present disclosure may be administered via oral or parenteral routes, and examples of parenteral administration may include intravenous administration, intraarterial administration, intrarectal administration, subcutaneous administration, intradermal administration, intramuscular administration, intranasal administration, mucosal administration, intradural administration, intraperitoneal administration, intraocular administration, and the like, and specifically, the pharmaceutical composition of the present disclosure may be administered intravenously.

The suitable dose of the pharmaceutical composition of the present disclosure varies depending on factors, such as a formulating method, a manner of administration, patient's age, body weight, gender, morbidity, and food, a time of administration, a route of administration, an excretion rate, and response sensitivity. The ordinarily skilled practitioners can easily determine and prescribe the dose that is effective for the desired treatment or prevention. According to an embodiment of the present disclosure, the daily dose of the pharmaceutical composition of the present disclosure is 0.001-100 mg/kg.

As for the dosage and frequency of administration of SN-38 and irinotecan hydrochloride contained in SNB-101 of the present disclosure, SN-38 may be administered at a dose of 1 to 5 mg/m2 per day once or in divided aliquots according to a specific embodiment of the present disclosure. For an injection, for example, a daily dose of 1 to 100 mg, specifically 20 mg to 85 mg, and more specifically 30 mg to 85 mg may be intravenously administered to a human patient (based on a body weight of 60 kg; body surface area 1.67 m2). The administration may be carried out in one to three divided doses, but with no limitations thereto.

The pharmaceutical composition of the present disclosure may be formulated into a unit dosage form or may be prepared in a multi-dose container by using a pharmaceutically acceptable carrier and/or excipient according to a method that is easily conducted by a person having an ordinary skill in the art to which the present disclosure pertains. Here, the dosage form may be a solution in an oily or aqueous medium, a suspension, an emulsion, an extract, a powder, granules, a tablet, or a capsule, and may further comprise a dispersant or a stabilizer.

The pharmaceutical composition of the present disclosure may be administered in parallel with a known compound or pharmaceutical composition having a cancer treatment effect.

In one embodiment of the present disclosure, the known compound or pharmaceutical composition includes at least one selected from the group consisting of a taxane-based anticancer agent, an albumin-bound taxane-based anticancer agent, a vascular endothelial growth factor (VEGF) inhibitor, and gemcitabine.

According to one aspect of the present disclosure, the present disclosure provides a method for treating cancer, the method comprising a step of administering, to a subject in need of administration, (a) a first pharmaceutical composition comprising particles comprising a hydrophobic camptothecin-based compound, a hydrophilic camptothecin-based compound, and an amphiphilic block copolymer composed of a hydrophobic block and a hydrophilic block in combination with (b) a second pharmaceutical composition comprising an antitumor agent as an active ingredient, or anticancer therapy.

In one embodiment of the present disclosure, the first pharmaceutical composition and the second pharmaceutical composition are administered simultaneously, separately, or sequentially.

In one embodiment of the present disclosure, the first pharmaceutical composition and the second pharmaceutical composition are administered as a combined formulation or a single formulation.

In one embodiment of the present disclosure, the anti-tumor therapy includes surgery, radiation therapy, or a combination thereof.

In one embodiment of the present disclosure, the cancer may be selected from the group consisting of gastric cancer, ovarian cancer, uterine cancer, cervical cancer, small cell lung cancer, non-small cell lung cancer, pancreatic cancer, breast cancer, esophageal cancer, retinoblastoma, oral cancer, salivary gland cancer, laryngeal cancer, pharyngeal cancer , rectal cancer, colon cancer, colorectal cancer, kidney cancer, prostate cancer, melanoma, liver cancer, gallbladder and other biliary tract cancer, thyroid cancer, bladder cancer, brain cancer, central nervous system cancer, bone tumor, skin cancer, non-Hodgkin's lymphoma, and Hodgkin's lymphoma, but with no limitations thereto. The brain cancer may be glioma, meningioma, schwannoma, a pituitary tumor, a metastatic brain tumor, or a base cranial tumor. The glioma includes astrocytoma, oligodendroglioma, ependymoma, or a mixture thereof.

In a specific embodiment of the present disclosure, the cancer is pancreatic cancer, stomach cancer, breast cancer, lung cancer, colorectal cancer, or a combination thereof.

Provided according to another aspect of the present disclosure are a pharmaceutical combination including: particles comprising a hydrophobic camptothecin-based compound, a hydrophilic camptothecin-based compound, and an amphiphilic block copolymer composed of a hydrophobic block and a hydrophilic block; and an antitumor agent as active ingredients.

In the pharmaceutical combination, each active ingredient is administered simultaneously, individually or sequentially for cancer treatment.

Another aspect of the present disclosure provides a kit for treating cancer, the kit comprising: a first pharmaceutical composition including particles comprising a hydrophobic camptothecin-based compound, a hydrophilic camptothecin-based compound, and an amphiphilic block copolymer composed of a hydrophobic block and a hydrophilic block; and a second pharmaceutical composition including an antitumor agent.

The first pharmaceutical composition and the second pharmaceutical composition in the kit for cancer treatment are administered simultaneously, separately, or sequentially for cancer treatment.

In an embodiment of the present disclosure, the first pharmaceutical composition and the second pharmaceutical composition are administered as a combined formulation or a single formulation.

As used herein, the term "administration" or "administer" refers to the direct application of a therapeutically effective amount of the composition of the present disclosure to a subject (i.e., an individual) with cancer, thereby forming the same amount thereof in the body of the subject.

The term "therapeutically effective amount" of the composition refers to the content of the composition, which is sufficient to provide a therapeutic or preventive effect to a subject to be administered, and thus the term has a meaning including "prophylactically effective amount." As used herein, the term "subject" includes, but is not limited to, human, mouse, rat, guinea pig, dog, cat, horse, cow, pig, monkey, chimpanzee, baboon, or rhesus monkey. Specifically, the subject of the present disclosure is human.

The method for treating cancer, the pharmaceutical combination preparation, and the kit for cancer treatment of the present disclosure include the same active ingredient as the pharmaceutical composition for the treatment of cancer according to an embodiment of the present disclosure, and employs an antitumor agent for a combination. Hence, the description given in the foregoing with respect to one aspect of the present disclosure is equally applied to the overlapping contents.

Advantageous Effects of Invention

The present disclosure relates to a use of nanoparticles comprising an poorly soluble camptothecin-based compound for treating cancer and an administration regimen in combination with a taxane-based antitumor agent. Exhibiting a synergistic cancer treatment effect when used in combination with a taxane-based antitumor agent, the pharmaceutical composition comprising the nanoparticles of the present disclosure can be easily used as a cancer treatment agent and as a combination therapy agent.

Brief Description of Drawings

FIG. 1 is a plot of mouse body weight changes with time after administration to pancreatic cancer model mice having AsPC-1 subcutaneously transplanted thereinto.

FIG. 2 is a plot of tumor volume changes with time in pancreatic cancer model mice having AsPC-1 subcutaneously transplanted thereinto, exhibiting inhibitory effects of test substances on tumors.

FIG. 3 is a graph of tumor weights in pancreatic cancer model mice having AsPC-1 subcutaneously transplanted thereinto, exhibiting inhibitory effects of test substances on tumors.

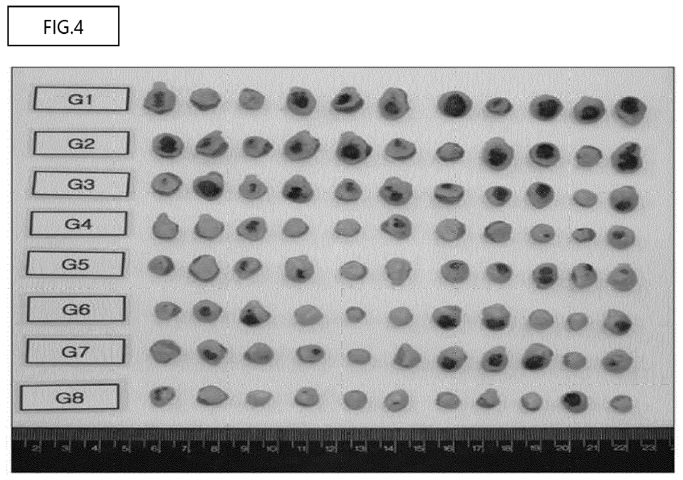

FIG. 4 is a photographic image of tumors excised on day 22 after administration of test substances to pancreatic cancer model mice having AsPC-1 subcutaneously transplanted thereinto.

FIG. 5 is a plot of mouse body weight changes with time after administration to gastric cancer model mice having Hs746T subcutaneously transplanted thereinto.

FIG. 6 is a plot of tumor volume changes with time in gastric cancer model mice having Hs746T subcutaneously transplanted thereinto, exhibiting inhibitory effects of test substances on tumors.

FIG. 7 is a graph of tumor weights in gastric cancer model mice having Hs746T subcutaneously transplanted thereinto, exhibiting inhibitory effects of test substances on tumors.

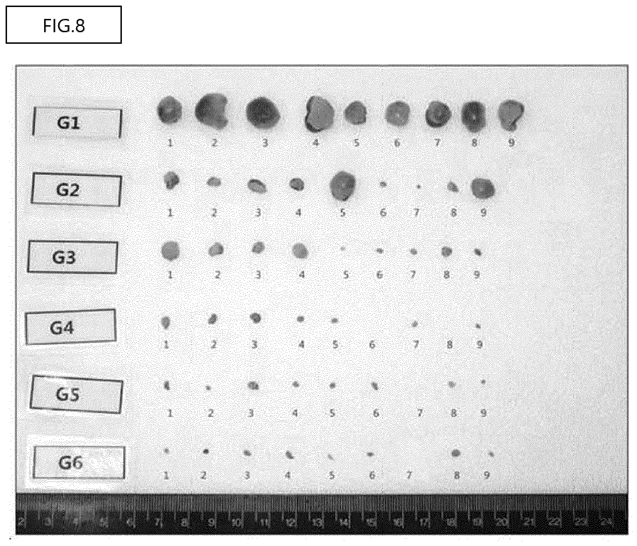

FIG. 8 is a photographic image of tumors excised on day 22 after administration of test substances to gastric cancer model mice having Hs746T subcutaneously transplanted thereinto.

FIG. 9 is a plot of tumor volume changes with time in breast cancer model mice having MDA-MB23 subcutaneously transplanted thereinto, exhibiting inhibitory effects of test substances on tumors.

FIG. 10 is a plot of mouse body weight changes with time after administration of test substances to breast cancer model mice having MDA-MB231 subcutaneously transplanted thereinto.

FIG. 11 is a plot of tumor volume changes with time in lung cancer model mice having A549 subcutaneously transplanted thereinto, exhibiting inhibitory effects of test substances on tumors.

FIG. 12 is a plot of mouse body weight changes with time in lung cancer model mice having A549 subcutaneously transplanted thereinto.

FIG. 13 is a plot of tumor volume changes with time after administration of test substances to colorectal cancer model mice having HT-29 subcutaneously transplanted thereinto.

FIG. 14 is a plot of tumor volume changes with time after administration of test substances to small cell lung cancer model mice having NCI-H69 subcutaneously transplanted thereinto.

FIG. 15 is a photographic image of tumors after administration of test substances to small cell lung cancer model mice having NCI-H69 subcutaneously transplanted thereinto.

FIG. 16 is a plot of tumor volume changes with time after administration of test substances to colorectal cancer model mice having HCT116 subcutaneously transplanted thereinto.

FIG. 17 is a photographic image of tumors after administration of test substances to colorectal cancer model mice having HCT116 subcutaneously transplanted thereinto.

FIG. 18 is a plot of tumor volume changes with time after administration of test substances to non-small cell lung cancer model mice having A549 subcutaneously transplanted thereinto.

Mode for Carrying out the Invention

Hereinafter, the present disclosure will be described in detail with reference to examples. These examples are only for illustrating the present disclosure more specifically, and it will be apparent to those skilled in the art that the scope of the present disclosure is not limited by these examples.

EXAMPLES

Throughout the present specification, the term "%" used to express the concentration of a specific material, unless otherwise particularly stated, refers to (wt/wt)% for solid/solid, (wt/vol)% for solid/liquid, and (vol/vol)% for liquid/liquid.

EXAMPLE 1: Preparation of Inventive Nanoparticles (SNB-101)

In a round-bottom flask, 10 ml of acetonitrile was added to 20 mg of SN-38 (7-ethyl-10-hydroxycamptothecin) as a hydrophobic camptothecin and 30 mg of irinotecan hydrochloride (trihydrate) as a hydrophilic camptothecin, followed by sonication in a sonicator to completely dissolve the two drugs. Next, 100 mg of methoxypolyethylene glycol-poly(D,L, lactic acid) [mPEG-Poly(D,L) lactic acid] (mPEG-PDLLA) was weighed in a round-bottom flask and completely dissolved in an organic solvent (ethanol-acetonitrile 50:50) v/v mixed solution) by stirring 30 minutes. The completely dissolved mPEG-PDLLA solution was slowly added several times to the drug solution in which SN-38 and irinotecan hydrochloride were dissolved, with stirring for 10 to 15 seconds each time. After the two solutions were mixed in a round-bottom flask, the organic solvent was completely removed by drying in a rotary evaporator to obtain a thin film. Then, 20ml of sterile distilled water was added to the film, followed by ultrasonication in a sonicator to completely dissolve the drug film.

The double-structure nanoparticles thus obtained were diluted to an SN-38 concentration of 1 mg/ml in a distilled water and measured for intensity weight-averaged diameter by dynamic light scattering using the Zetasizer Nano System of Malvern (UK).

Next, 200 mg of trehalose and 300 mg of mannitol (D-Mannitol) as cryoprotectants were completely dissolved in 20 ml of sterile distilled water by stirring.

Finally, all the cryoprotectant solution was added to the nanoparticle solution of the mixed drugs while stirring for 10 to 15 seconds each time. The final mixed solution was filtered through a 0.22 um cellulose acetate membrane filter and loaded into packaging vials. The filled vials were lyophilized to obtain a finished product in the form of a dry powder or cake (named SNB-101).

EXAMPLE 2: Effect of Co-Administration of Nanoparticles (SNB-101) on Pancreatic Cancer

2-1. Experimental material

As substances for the test, a 5% glucose injection solution, irinotecan hydrochloride (irinotecan HCl; CalmtopTM Inj. CJ Healthcare, Korea), nab-paclitaxel (AbraxaneTM, Abraxis BioScience, LLC), and a nanoparticle composition (designated as SNB-101) comprising SN-38 and irinotecan hydrochloride were used. The irinotecan hydrochloride (Calmtop TM Inj.) was 100 mg/5 mL and was purchased from CJ Healthcare. Abraxane was purchased from Abraxis BioScience, LLC (USA) and used as a lyophilized injection with 100 mg/vial.

The nanoparticle composition (SNB-101) comprising SN-38 and irinotecan hydrochloride, used in the present disclosure, is a stable nanoparticle formulation as an injection, and the preparation method therefor is introduced in Korean Patent No. 10-2094543. The intravenous injection of SNB-101 contained 10 mg of SN-38 and 15.9 mg of irinotecan hydrochloride per vial. Before use, the injection may be diluted in 9.64 mL of 5% dextrose injection USP, and the dilution may be injected over 90 minutes.

2-2. Test method

1) AsPC-1 cell culture

As a pancreatic cancer cell line for a pancreatic cancer xenograft model, AsPC-1 was used. AsPC-1 cells are aggressive cells and are known to have resistance to gemcitabine. AsPC-1 cells were cultured in Roswell Park Memorial Institute medium (RPMI-1640) supplemented with 10% fetal bovine serum (FBS) and 1% penicillin/streptomycin in a 175-cm2 flask. FBS, RPMI-1640, and Penicillin-Streptomycin were purchased from ATCC (Manassas, VA) and used in the experiment. The cells were cultured at 37°C in a 5% CO2 incubator. Each cultured cancer cell was re-fed 2-3 times a week and washed with phosphate buffered saline (PBS, pH 7.4). Then, the attached cells were detached with 0.05% trypsin-0.02% EDTA. The separated cells were centrifuged (3 min, 1500 rpm). The pellet of the cancer cells was added with the medium and suspended by pipetting. After passages, the cells were used in the experiment.

2) Induction of pancreatic cancer xenograft mouse animal model

The animals used in this test were BALB/c nu/nu male mice (about 5 weeks old, average weight 19g±20%), which are widely used for xenotransplantation animal models, and acclimated for about 5-7 days after being purchased. On the day of inoculation, the cultured cancer cells were washed with phosphate buffered saline (PBS, pH 7.4), and trypsinized with 0.05% Trypsin-0.02% EDTA to detach the cells. The cells were harvested by briefly spinning (3 minutes, 1500 rpm), diluted in PBS, and then subcutaneously injected at a dose of 5x106 cells/0.2 mL per animal into the right flank of the mice, and it was confirmed that the tumor cell suspension did not leak from the injection site.

Grouping and dosage setting

The mice were grouped based on the average pancreatic cancer tumor volume of 200±20 mm3, and the control and test groups and dosages therefor were as follows.

TABLE1

| Group | Control/Test Group | | Dose (mg/kg/ day) | Count |
|---|---|---|---|---|
| G1 | Vehicle | 5% Glucose injection | - | 11 |
| G2 | Positive control 1 | Nab-paclitaxel | 5 | 11 |
| G3 | Positive control 2 | Irinotecan HCl | 50 | 11 |
| G4 | Positive control 3 | Nab-paclitaxel + Irinotecan HCl | 5 + 50 | 11 |
| G5 | Test group 1 (low-dose) | SNB-101 | 10/15.9 * | 11 |
| G6 | Test group 2 (high-dose) | SNB-101 | 20/31.8 * | 11 |
| G7 | Test group 3 (combined, low dose) | Nab-paclitaxel + SNB-101 | 5 + 10/15.9 * | 11 |
| G8 | Test group 4 (combined, high dose) | Nab-paclitaxel + SNB-101 | 5 + 20/31.8 * | 11 |
| *: dose for SN-38/irinotecan HCl in SNB-101 | | | | |

3) Injection site and injection mode

The test substances were injected via the tail vein (intravenous, i.v.) at respective doses calculated on the basis of the weights of the mice just before administration. The test substances were administered once a week, a total of 5 times (days 0, 7, 14, 17, and 21), and autopsy was performed on day 22.

4) Observation and examination items

Clinical symptoms were observed once a day during the test period, and along drug administration, the tumor volume was measured three times a week. After completion of the test, the animals were anesthetized with isoflurane and then exsanguinated. Tumor tissues were excised for each individual. The excised tumors were weighed and photographed. Long and short diameters of tumor were measured using vernier calipers (Mitutoyo Corp., Model No.: CD-15CPX, Japan), and the tumor volume was calculated according to Equation 1, below.

$$\text{Equation 1}$$
$$\text{Tumor volume} = (\text{short axis})2 \times \text{long axis} \times 0.5$$

5) Statistics

The test results were statistically analyzed through Mann-Whitney test using a commercial statistical program (IBM SPSS statistics version 19.0).

2-3. Test results

1) Body weight

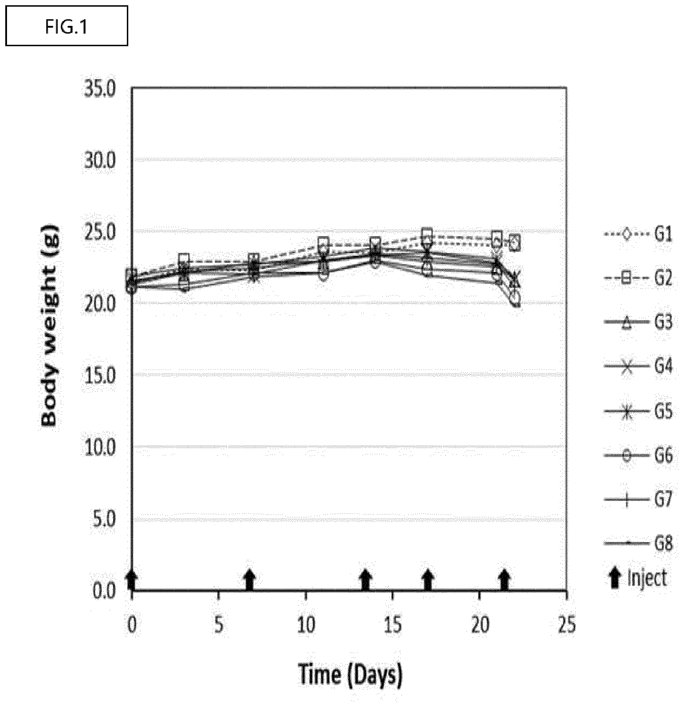

Body weight measurements on the 22nd day (the day of autopsy) of administration of the test substance are summarized in Table 2 and FIG. 1.

TABLE 2

| Gro up | Day 0 | Day 3 | Day 7 | Day 11 | Day 14 | Day 17 | Day 21 | Day 22 |
|---|---|---|---|---|---|---|---|---|
| G1 | 21.5±1.1 | 22.3±1.0 | 22.3±1.1 | 23.6±1.1 | 23.5±1.3 | 24.2±1.1 | 24.1±1.0 | 24.1±1.0 |
| G2 | 21.9±1.0 | 23.0±1.0 | 22.9±0.7 | 24.0±0.9 | 24.1±0.9 | 24.6±1.0 | 24.5±1.0 | 24.2±1.1 |
| G3 | 21.3±0.9 | 22.1±1.1 | 22.8±1.1 | 22.9±1.2 | 23.4±1.2 | 22.9±1.2 | 2 2.6±1.2 | 21.7±1.3 |
| G4 | 21.9±0.7 | 22.5±0.9 | 22.7±1.0 | 23.3±1.2 | 23.9±1.2 | 23.6±1.1 | 23.1±1.1 | 21.7±1.2 |
| G5 | 21.6±1.5 | 22.2±1.6 | 22.1±1.5 | 23.0±1.4 | 23.4±1.5 | 23.5±1.7 | 22.8±1.6 | 21.7±1.5 |
| G6 | 21.2±1.2 | 21.3±1.3 | 22.1±1.1 | 22.1±1.2 | 23.0±1.1 | 22.4±1.2 | 22.1±1.0 | 20.4±0.9 |
| G7 | 21.5±1.1 | 22.2±0.9 | 22.5±0.8 | 23.0±1.0 | 23.4±1.0 | 23.2±1.2 | 22.7±1.2 | 21.1±1.2 |
| G8 | 21.2±1.4 | 21.0±1.8 | 21.8±1.8 | 22.1±1.7 | 22.9±1.5 | 22.0±1.4 | 21.4±1.2 | 19.9±1.1 |

Compared to G1 (vehicle group), the test substance administration groups (G3-G8) exhibited weight loss rates of 10.0%, 10.0%, 10.0%, 15.4%, 12.4%, and 17.4%, respectively, which were all under the critical weight loss rate 20% (FIG. 1).

2) Measurements of tumor volume

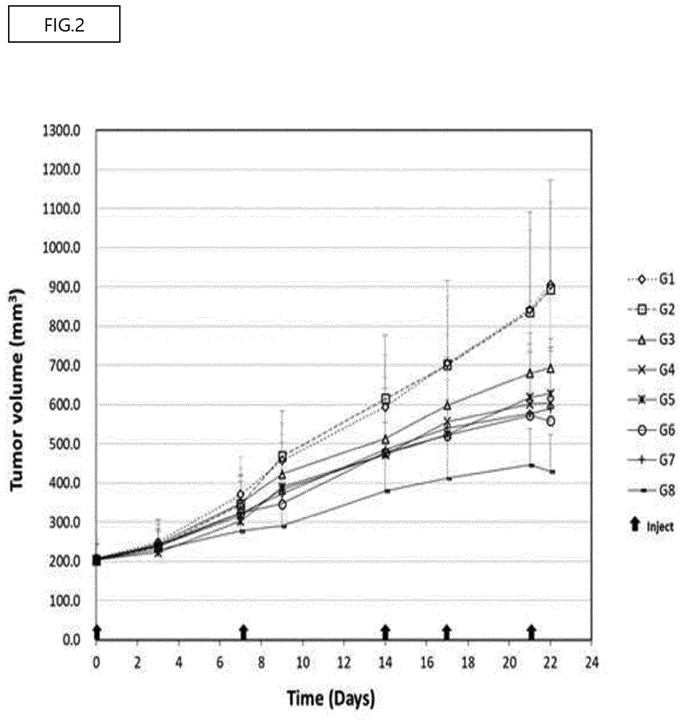

Measurements of pancreatic cancer tumor volumes are summarized in Table 3 and depicted in FIG. 2.

TABLE 3

| Gro up | 0 day | 3 days | 7 days | 9 days | 14 day | 17 days | 21 days | 22 days |
|---|---|---|---|---|---|---|---|---|
| G1 | 208.±156.5 | 249.4±57.1 | 370.4±67.8 | 457.9±94.4 | 594.4±132.0 | 705.3±169.2 | 843.1±201.8 | 906.7±210.9 |
| G2 | 202.3±53.8 | 237.0±39.6 | 346.3±74.9 | 467.9±117.4 | 614.7±163.6 | 701.8±213.2 | 837.7±253.2 | 895.2±276.6 |
| G3 | 205.0±50.2 | 244.2±62.6 | 349.5±116.4 | 423.3±100.8 | 513.3±155.9 | 599.0±178.5 | 681.0±183.8 | 693.8±180.5 |
| G4 | 206.8±48.5 | 238.5±65.7 | 316.7±87.7 | 383.2±119.1 | 472.2±136.3 | 556.4±165.0 | 602.0±180.8 | 605.0±164.3 |
| G5 | 203.4±40.5 | 222.8±33.0 | 302.8±61.9 | 389.9±77.5 | 473.9±79.8 | 525.2±88.1 | 618.3±115.8 | 628.0±108.5 |
| G6 | 202.3±39.8 | 239.0±55.2 | 323.9±93.9 | 347.3±121.2 | 477.8±162.9 | 522.3±178.8 | 572.4±181.8 | 558.9±186.8 |
| G7 | 203.5±41.2 | 243.2±41.9 | 325.2±63.6 | 371.9±94.0 | 486.2±128.8 | 540.5±122.6 | 577.0±160.8 | 590.5±153.1 |
| G8 | 204.4±42.7 | 229.9±52.8 | 277.6±71.4 | 289.9±75.6 | 379.8±87.0 | 412.1±96.1 | 445.6±93.7 | 430.0±93.6 |

The volume of the pancreatic cancer tumor continued to increase in the vehicle group (G1) and the nab-paclitaxel alone group (G2; 5 mg/kg) until 22 days after group separation, with the tumor growth higher than those of the other groups.

Compared to the vehicle group, the tumor volume significantly decreased in the nab-paclitaxel + SNB-101 (high dose) group (G8) from day 7, and in all the SNB-101 (low, high dose) alone groups (G5, G6) and nab-paclitaxel + SNB-101 (high-dose) combination group (G8) from day 17 to day 22 ($p < 0.05$).

On day 22, the nab-paclitaxel + irinotecan hydrochloride combination group (G4) decreased in tumor volume by 32.4% compared to the nab-paclitaxel alone group (G2), and by 12.8% compared to the irinotecan hydrochloride alone group (G3), but with no statistical significance observed ($p > 0.05$).

Upon comparison between SNB-101 (low- and high-dose) alone groups (G5 vs. G6), the high-dose group (G6) significantly decreased in tumor growth by 11.0% compared to the low-dose group (G5), exhibiting that SNB-101 inhibited in a dose-dependent manner (10/15.9 mg/kg vs. 20/31.8 mg/kg as SN-38/Irinotecan HCl).

Upon comparison between the nab-paclitaxel alone group (G2) or SNB-101 (low- and high-dose) alone groups (G5 and G6) and the nab-paclitaxel + SNB-101 (low- and high dose) combination groups (G7 and G8), the two combination groups both showed higher tumor growth inhibitory effects than each group alone ($p < 0.05$, $p < 0.01$). In the comparison between nab-paclitaxel + SNB-101 (low and high dose) groups (G7 and G8), the tumor growth inhibitory effect also increased with statistical significance ($p < 0.05$) as the dose of SNB-101 increased.

Nab-paclitaxel (5mg/kg) alone (G2) could not confirm the tumor inhibition effect, but a higher inhibitory effect on tumor growth was observed in the nab-paclitaxel + SNB-101 high-dose group (G8) (20/31.9 mg/kg as SN-38/irinotecan HCl) (52.6%) than in the SNB-101 high-dose alone group (G6) (38.4%). In addition, the nab-paclitaxel + SNB-101 high-dose group (G8) showed a tumor growth inhibitory effect (29.5%), compared to the nab-paclitaxel + irinotecan HCl combination group (G4), with statistical significance ($p < 0.05$).

These results show that co-administration of nab-paclitaxel and SNB-101 can exhibit a synergistic inhibitory effect on pancreatic tumor growth.

3) Measurement of tumor weight

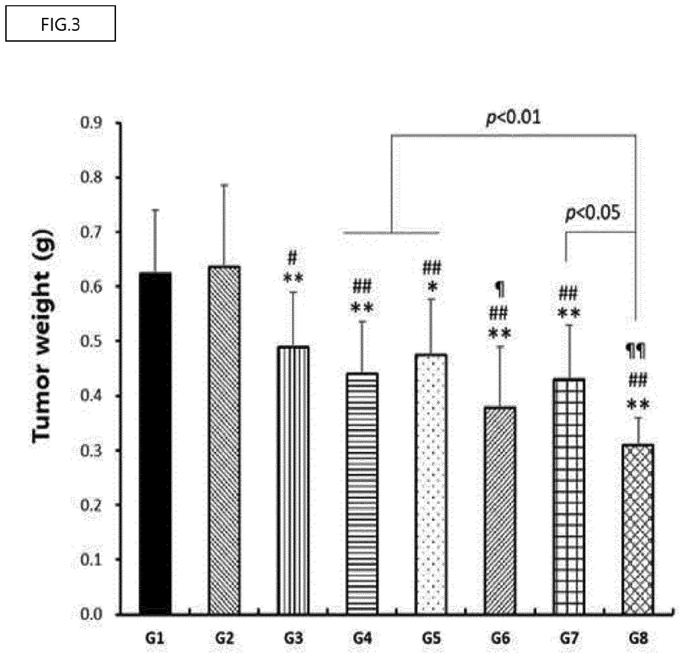

Measurements of tumor weight are summarized in Table 4, below, and depicted in FIG. 3.

TABLE 4

| Group | Tumor weight (g) |
|---|---|
| G1 | 0.623±0.117 |
| G2 | 0.636±0.150 |

(continued)

| Group | Tumor weight (g) |
|---|---|
| G3 | 0.489±0.101 |
| G4 | 0.440±0.096 |
| G5 | 0.474±0.102 |
| G6 | 0.379±0.112 |
| G7 | 0.431±0.097 |
| G8 | 0.310±0.050 |

As shown in Table 4 and FIG. 3, the average tumor weight was 0.62±0.11 g for the untreated group (vehicle group, G1), 0.64±0.15 g for the nab-paclitaxel treated group (G2), and 0.47±0.10g and 0.3810.11g for SNB-101 (low and high dose) alone groups (G5 and G6), respectively. In addition, the tumor weights of the nappaclitaxel + SNB-101 (low and high dose) combination groups (G7 and G8) were 0.43±0.10 and 0.31±0.05 g, respectively. The combination groups showed higher tumor inhibition by more than 50% than the nab-paclitaxel alone group (G2) and by 10-22.5% than SNB-101 (low and high dose) alone groups (G5 and G6). From the above results, it was understood that nab-paclitaxel and SNB-101 have the potential as an excellent combination therapy for pancreatic cancer treatment.

The nab-paclitaxel + irinotecan hydrochloride combination group (G4) significantly decreased by 30.8% compared to the nappaclitaxel alone group (G2). In comparison between SNB-101 (low and high dose) alone groups, the high-dose group (G6) decreased by 20.0% compared to the low-dose group (G5).

EXAMPLE 3: Effects of Co-Administration of Nanoparticles (SNB-101) on Gastric Cancer

3-1. Experimental materials

As substances for the test, a 5% glucose injection solution, irinotecan hydrochloride (irinotecan HCl; CalmtopTM Inj., CJ Healthcare, Korea), docetaxel (TaxotereTM, Sanofi-Aventis Korea), and a nanoparticle composition (designated as SNB-101) comprising SN-38 and irinotecan hydrochloride were used. The irinotecan hydrochloride (CalmtopTM Inj.) was 100 mg/5 mL and was purchased from CJ Healthcare. Docetaxel was purchased from Sanofi-Aventis Korea and used as a lyophilized injection with 20 mg/vial. The nanoparticle composition (SNB-101) comprising SN-38 and irinotecan hydrochloride, used in the present disclosure, is a stable nanoparticle formulation as an injection, and the preparation method therefor is introduced in Korean Patent No. 10-2094543. The intravenous injection of SNB-101 contained 10 mg of SN-38 and 15.9 mg of irinotecan hydrochloride per vial. Before use, the injection may be diluted in 9.64 mL of 5% dextrose injection USP, and the dilution may be injected over 90 minutes.

3-2. Test method

1) Culture of gastric cancer cell line Hs746T

As a gastric cancer cell line for a gastric cancer xenograft model, Hs746T was used. Hs746T cells were cultured in Dulbecco's modified eagle's medium (DMEM) supplemented with 10% fetal bovine serum (FBS) and 1% penicillin/streptomycin in a 175-cm2 flask. FBS, DMEM, and Penicillin-Streptomycin were purchased from ATCC (Manassas, VA) and used in the experiment. The cells were cultured at 37°C in a 5% CO2 incubator. Each cultured cancer cell was re-fed 2-3 times a week and washed with phosphate buffered saline (PBS, pH 7.4). Then, the attached cells were detached with 0.05% trypsin-0.02% EDTA. The separated cells were centrifuged (3 min, 1500 rpm). The pellet of the cancer cells was added with the medium and suspended by pipetting. After passages, the cells were used in the experiment.

On the day of inoculation, the cultured cancer cells were washed with PBS and detached by treatment with 0.05% Trypsin-0.02% EDTA. The detached cells were harvested by briefly spinning (3 min, 1500 rpm) and diluted in PBS to prepare a dose of 4x106 cells/0.2 mL per animal.

2) Induction of gastric cancer xenograft mouse animal model

The animals used in this test were BALB/c nu/nu male mice (about 5 weeks old, average weight 19g±20%), which are widely used for xenotransplantation animal models, and acclimated for about 5-7 days after being purchased.

On the day of inoculation, the cultured cancer cells were washed with phosphate buffered saline (PBS, pH 7.4), and trypsinized with 0.05% Trypsin-0.02% EDTA to detach the cells. The cells were harvested by briefly spinning (3 minutes, 1500 rpm), diluted in PBS, and then subcutaneously injected at a dose of 5x106 cells/0.2 mL per animal into the right flank of the mice, and it was confirmed that the tumor cell suspension did not leak from the injection site.

Grouping and dosage setting

The mice were grouped based on the average gastric cancer tumor volume of 80±20 mm3, and the control and test groups and dosages therefor were as follows.

TABLE 5

| Group | Control/Test group | | Dose (mg/kg/day) | Count |
|---|---|---|---|---|
| G1 | Vehicle | 5% Glucose injection | - | 9 |
| G2 | Positive control 1 | Docetaxel | 5 | 9 |
| G3 | Positive control 2 | Irinodecan HCl | 60 | 9 |
| G4 | Positive control 3 | Docetaxel + Irinodecan HCl | 5 + 60 | 9 |
| G5 | Test group 1 (alone) | SNB-101 | 20/31.8 * | 9 |
| G6 | Test group 2 (combination) | Docetaxel + SNB-101 | 5 + 20/31.8 * | 9 |
| * * dose for SN-38/irinotecan HCl in SNB-101 | | | | |

3) Injection site and injection mode

The test substances were injected via the tail vein (intravenous, i.v.) at respective doses calculated on the basis of the weights of the mice just before administration. The test substances were administered once a week, a total of 3 times (days 0, 7, and 14), and autopsy was performed on day 15.

4) Observation and examination items

Clinical symptoms were observed once a day during the test period, and along drug administration, the tumor volume was measured three times a week. After completion of the test, the animals were anesthetized with isoflurane and then exsanguinated. Tumor tissues were excised for each individual. The excised tumors were weighed and photographed. Long and short axes of tumor were measured using vernier calipers (Mitutoyo Corp., Model No.: CD-15CPX, Japan), and the tumor volume was calculated according to Equation 1, below.

```
Equation 1
Tumor volume = (short axis)2 x long axis x 0.5
```

5) Statistics

The test results were statistically analyzed through Mann-Whitney test using a commercial statistical program (IBM SPSS statistics version 19.0).

3-3. Test results

1) Body weight

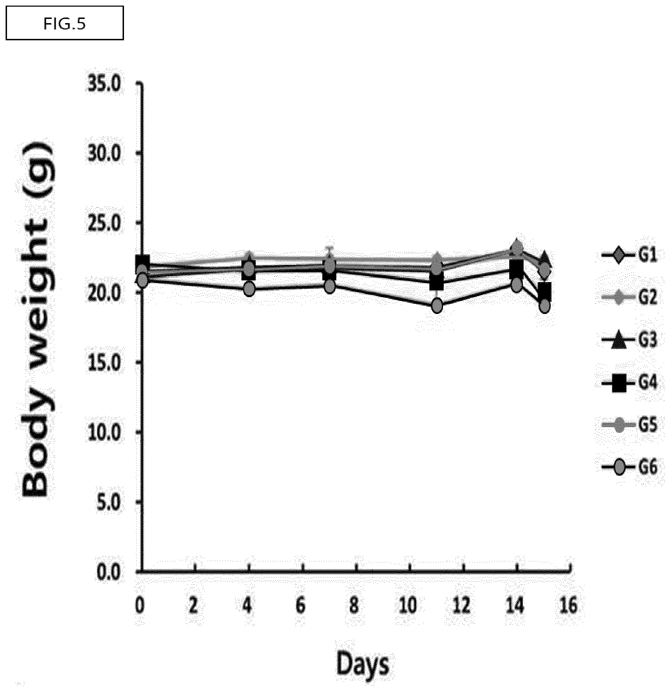

Body weight measurements on day 15 (the day of autopsy) of administration of the test substance are summarized in Table 6 and FIG. 5.

TABLE 6

| Group | 0 days | 4 days | 7 days | 11 day | 14 days | 15 days |
|---|---|---|---|---|---|---|
| G1 | 21.2±1.2 | 21.6±1.0 | 21.7±1.1 | 21.7±1.1 | 22.7±1.3 | 22.2±1.1 |

(continued)

| Group | 0 days | 4 days | 7 days | 11 day | 14 days | 15 days |
|---|---|---|---|---|---|---|
| G2 | 21.9±1.6 | 22.5±1.6 | 22.4±1.3 | 22.3±1.5 | 22.8±1.4 | 22.2±1.4 |
| G3 | 21.1±1.3 | 21.7±1.1 | 21.7±1.1 | 21.8±1.1 | 22.8±1.1 | 21.9±1.1 |
| G4 | 22.0±0.9 | 21.4±0.7 | 21.7±0.7 | 20.8±0.8 | 21.6±1.2 | 20.1±1.1 |
| G5 | 21.5±1.2 | 21.8±1.0 | 21.9±0.8 | 21.8±1.3 | 23.1±1.5 | 21.5±1.3 |
| G6 | 21.0±1.6 | 20.3±2.5 | 20.6±2.6 | 19.1±1.6 | 20.7±1.9 | 19.1±1.9 |

Unit: g, Data are expressed in mean±SD (n=9)
G1: Vehicle control (5% glucose injection)
G2: Positive control 1 (5 mg/kg/day Docetaxel)
G3: Positive control 2 (60 mg/kg/day Irinotecan HCl)
G4: Positive control 3 (5 mg/kg/day Docetaxel + 60 mg/kg/day Irinotecan HCl)
G5: Test article 1 (20/31.8 mg/kg/day (as SN-38/Irinotecan HCl) SNB-101)
G6: Test article 2 (5 mg/kg/day Docetaxel + 20/31.8 mg/kg/day (as SN-38/Irinotecan HCl) SNB-101)

When the mice were purchased and grouped, no significant difference in body weight was observed thereamong. On day 15 (the day of autopsy) of administration of the test substances, weight loss was observed in the groups G4 (Docetaxel + Irinotecan HCl) and G6 (Docetaxel + SNB-101). On day 15, the combined groups were observed to significantly decrease in body weight, compared to all the vehicle control group G1 (5% glucose injection) and the single groups G2 (Docetaxel), G3 (Irinotecan HCl), and G4 (SNB-101). Any of the test substance-administered groups (G2-G6) did not exhibit a weight loss rate of more than 20%, compared to G1 (vehicle group), indicating that no test groups reached the critically low body weight (FIG. 5).

2) Measurements of tumor volume

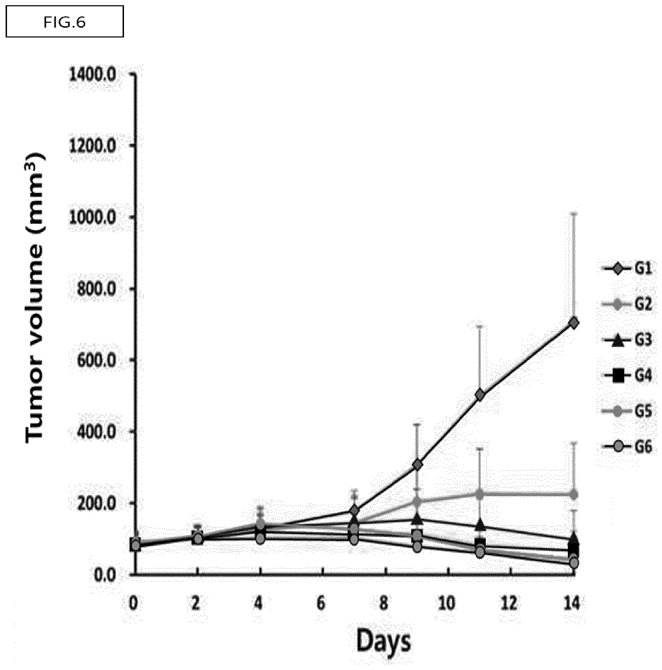

Measurements of pancreatic cancer tumor volumes are summarized in Table 7 and depicted in FIG. 6.

TABLE 7

| Grou p | 0 day | 2 days | 4 days | 7 days | 9 day | 11 days | 14 days |
|---|---|---|---|---|---|---|---|
| G1 | 84.0±1 9.6 | 98.9±2 5.0 | 129.9±28.8 | 178.2±4 4.7 | 305.6±1 09.3 | 499.8±19 0.8 | 708.2±30 8.8 |
| G2 | 89.6±2 2.1 | 99.8±2 1.6 | 127.2±40.1 | 146.0±8 0.4 | 203.5±1 09.3 | 227.1±13 6.3** | 224.8±15 8.3** |
| G3 | 81.7±2 6.5 | 100.0±37.2 | 127.5±56.5 | 145.8±6 7.7 | 155.7±8 3.9* | 133.3±79 .7** | 99.4±80. 8** |
| G4 | 84.4±2 8.1 | 99.3±3 3.9 | 121.5±41.7 | 110.2±4 7.3** | 116.2±4 4.8** | 83.9±46. 2**# | 67.9±51. 6**# |
| G5 | 86.0±2 5.0 | 105.2±24.9 | 142.5±39.9 | 127.7±4 2.5* | 108.8±4 2.4** | 70.1±37. 7**## | 41.9±13. 9**# |
| G6 | 92.4±2 3.6 | 100.5±31.8 | 103.5±41.9 | 101.5±3 7.1** | 79.2±30 . 0***#¶ | 62.9±26. 8***#¶ | 30.5±15. 1**##¶¶ |

Unit: g, data are expressed in mean±SD (n=9)
* Compared with G1: *p<0.05, **p<0.01; # Compared with G2: # p<0.05, ## p<0.01; ¶ Compared with G3: ¶ p<0.05, ¶¶ p<0.01
G1: Vehicle control (5% glucose injection)
G2: Positive control 1 (5 mg/kg/day Docetaxel)
G3: Positive control 2 (60 mg/kg/day Irinotecan HCl)
G4: Positive control 3 (5 mg/kg/day Docetaxel + 60 mg/kg/day Irinotecan HCl)
G5: Test article 1 (20/31.8 mg/kg/day (as SN-38/Irinotecan HCl) SNB-101)
G6: Test article 2 (5 mg/kg/day Docetaxel + 20/31.8 mg/kg/day (as SN-38/Irinotecan HCl) SNB-101)

The volume of the gastric cancer tumor continued to increase in the vehicle group (G1) and the nab-paclitaxel alone group (G2; 5 mg/kg) until 22 days after group separation, with the tumor growth higher than those of the other groups.

Compared to the vehicle control group G1, the tumor volume significantly decreased in G4 (Docetaxel + Irinotecan HCl combination), G5 (SNB-101 alone) and G6 (Docetaxel + SNB-101 combination) from day 7, and in all the positive groups (G2, G3, and G4) and test substance groups (G5 and G6) on day 15.

From day 11, G4 (Docetaxel + Irinotecan HCl combination group) decreased in tumor volume, compared to G2 (Docetaxel alone group), with statistical significance, and G6 (Docetaxel + SNB-101 combination group) significantly decreased in tumor volume from day 8, compared to G2 (Docetaxel alone group) and G3 (Irinotecan HCl alone group).

3) Measurement of tumor weight

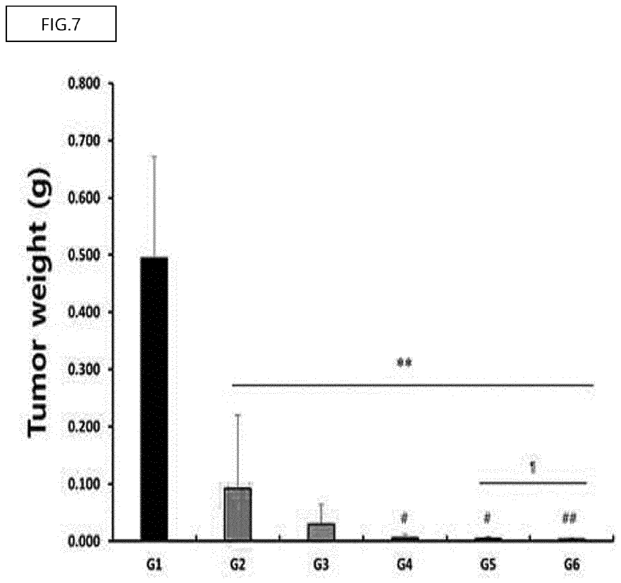

Measurements of tumor weight are summarized in Table 8, below, and depicted in FIG. 7.

TABLE 8

| Group | Tumor weight (g) |
|---|---|
| G1 | $0.494 \pm 0.178$ |
| G2 | $0.092 \pm 0.128$** |
| G3 | $0.029 \pm 0.035$** |
| G4 | $0.006 \pm 0.006$*** |
| G5 | $0.004 \pm 0.003$**#¶ |
| G6 | $0.003 \pm 0.003$**##¶ |
| Data are expressed in mean$\pm$SD (n=9); <br> *Compared with G1: ** $p<0.01$; <br> #Compared with G2: # $p<0.05$, ## $p<0.01$; <br> ¶Compared with G3: ¶ $p<0.05$ <br> G1: Vehicle control (5% glucose injection) <br> G2: Positive control 1 (5 mg/kg/day Docetaxel) <br> G3: Positive control 2 (60 mg/kg/day Irinotecan HCl) <br> G4: Positive control 3 (5 mg/kg/day Docetaxel + 60 mg/kg/day Irinotecan HCl) <br> G5: Test article 1 (20/31.8 mg/kg/day (as SN-38/Irinotecan HCl) SNB-101) <br> G6: Test article 2 (5 mg/kg/day Docetaxel + 20/31.8 mg/kg/day (as SN-38/Irinotecan HCl) SNB-101) | |

The tumors were excised and weighed, and as shown in Table 8 and FIG. 7, the tumor weight was significantly decreased in all the positive groups (G2, G3, and G4), the test alone group(G5), and the combination group (G6), compared to the vehicle control G1. Significant tumor weight decreases were also observed G4 (Docetaxel + Irinotecan HCl combination), G5 (SNB-101 alone) and G6 (Docetaxel + SNB-101 combination), compared to G2 (Docetaxel alone). In addition, the SNB-101 alone group and the Docetaxel + SNB-101 combination group exhibited significant decrease in tumor weight, compared to the Irinotecan HCl alone group.

As demonstrated in the xenograft models using the gastric cancer cell line Hs746T under the test condition, the positive substances G2 (5 mg/kg/day Docetaxel), G3 (60 mg/kg/day Irinotecan HCl alone), G4 (5 mg/kg/day Docetaxel + 60 mg/kg/day Irinotecan HCl combination) and the test substances G5 (20/31.8 mg/kg/day (as SN-38/Irinotecan HCl)) SNB-101 alone) and G6 (5 mg/kg/day Docetaxel + 20/31.8 mg/kg/day (as SN-38/Irinotecan HCl) SNB-101 combination) were all effective for inhibiting tumor growth, with higher inhibitory activity in the combination groups (G4 and G6) than the positive control alone groups (G2 and G3).

EXAMPLE 4: Effect of Co-Administration of Nanoparticle (SNB-101) on Breast Cancer and Lung Cancer

4-1. Experimental material

As substances for the test, a 5% glucose injection solution, docetaxel (Taxotere™, Sanofi-Aventis Korea), and a nanoparticle composition (designated as SNB-101) comprising SN-38 and irinotecan hydrochloride were used. Docetaxel was purchased from Sanofi-Aventis Korea and used as a lyophilized injection with 20 mg/vial.

The nanoparticle composition (SNB-101) comprising SN-38 and irinotecan hydrochloride, used in the present disclosure, is a stable nanoparticle formulation as an injection, and the preparation method therefor is introduced in

**EP 4 252 747 A1**

Korean Patent No. 10-2094543. The intravenous injection of SNB-101 contained 10 mg of SN-38 and 15.9 mg of irinotecan hydrochloride per vial. Before use, the injection may be diluted in 9.64 mL of 5% dextrose injection USP, and the dilution may be injected over 90 minutes.

4-2. Test method

1) Culture of breast cancer cell line (MDA-MB-231) and lung cancer cell line (A549)

As breast cancer and lung cancer cell lines for xenograft models, MDA-MB-231 and A549 were used, respectively. Frozen MDA-MB-231 and A549 cells were thawed and cultured in RPMI1640 medium-10% FBS-1% Penicillin-Streptomycin and RPMI1640 medium-5% FBS-1%

Penicillin-Streptomycin, respectively, at 37°C in a 5% CO2 condition. The thawed cells were cultured for one week or longer, with passages every two or three days. The cells were assayed for viability (trypan blue) and infection with bacteria and yeasts (cell images) and mycoplasma (MycoAlert Mycoplasma detection kit). Used for transplantation were the cells that were not infected and exhibited a viability of 90% or higher as measured by trypan blue staining.

2) Induction of breast and lung cancer xenograft mouse animal model

The animals used in this test were BALB/c nu/nu male mice (about 5 weeks old, average weight 19g±20%), which are widely used for xenotransplantation animal models, and acclimated for about 5-7 days after being purchased. On the day of inoculation, the cultured cancer cells were washed with phosphate buffered saline (PBS, pH 7.4), and trypsinized with 0.05% Trypsin-0.02% EDTA to detach the cells. The cells were harvested by briefly spinning (3 minutes, 1500 rpm), diluted in PBS, and then subcutaneously injected at a dose of 5x106 cells/100 $\mu l$ per animal into the right flank of the mice, and it was confirmed that the tumor cell suspension did not leak from the injection site.

3) Grouping and dosage, injection site, and injection mode setting

The mice were grouped, based on the average gastric cancer tumor volume of 90-105 mm3, using the paired-matching method, and the control and test groups and dosages therefor are summarized in Tables 9 and 10.

TABLE 9 Breast Cancer Cell Line MDA-MB-231

| Group No . | Drug | N | Dose (mg/kg) | Route | Treatment Frequency |
|---|---|---|---|---|---|
| G1 | Vehicle | 10 | - | i.v. | Q1W x 6** |
| G2 | Docetaxel | 10 | 5 -> 15* | i.v. | Q1W x 6** |
| G3 | SNB-101 | 10 | 20*** | i.v. | Q1W x 6** |
| G4 | SNB-101+ docetaxel | 10 | 20***5 - > 15* | i.v. | Q1W x 6** |

* Docetaxel administration concentration was changed from 5 mg/kg (1st to 3rd doses) to 15 mg/kg (4th to 6th doses) during the test.
** The frequency of drug administration for all groups was changed from once a week for 3 weeks (Q1W x 3 times) to once a week for 6 weeks (Q1W x 6 times) during the test.
*** The dose of 20 mg/kg of SNB-101 was based on the content of SN-38.

TABLE 10 Lung Cancer Cell Line A549

| Group No. | Drug | N | Dose (mg/kg) | Route | Treatment Frequency |
|---|---|---|---|---|---|
| G1 | Vehicle | 10 | - | i.v. | Q1W x 5** |
| G2 | Docetaxel | 10 | 5 -> 10* | i.v. | Q1W x 5** |
| G3 | SNB-101 | 10 | 20 | i.v. | Q1W x 5** |

(continued)

| Group No. | Drug | N | Dose (mg/kg) | Route | Treatment Frequency |
|---|---|---|---|---|---|
| G4 | SNB-101+ docetaxel | 10 | 205 -> 10* | i.v. | Q1W x 5** |

* Docetaxel administration concentration was changed from 5 mg/kg (1st dose) to 10 mg/kg (2nd to 5th doses) during the test.
** The frequency of drug administration for all groups was changed from once a week for 3 weeks (Q1W x 3 times) to once a week for 5 weeks (Q1W x 5 times) during the test.
*** The dose of 20 mg/kg of SNB-101 was based on the content of SN-38.

Tumor volume and body weight were measured twice a week (intervals of 3 or 4 days) from the time of grouping to the end of the test, and a tumor growth curve was constructed from the start of drug administration to the end of the test.

When severe necrosis and weight loss were found, the test was terminated by euthanasia in accordance with the Animal Experimental Ethics Committee (IACUC) regulations in consultation with the sponsor, and the tumor was excised and imaged.

4-3. Breast cancer MDA-MB-231 xenograft model test result

1) Tumor volume measurement and result analysis

Compared to G1 (vehicle), tumor growth inhibitory efficacy of G2 (docetaxel alone) or G3 (SNB-101 alone) and G4 (SNB-101 + docetaxel combination) groups were measured on the end of the experiment (35 days after drug administration).

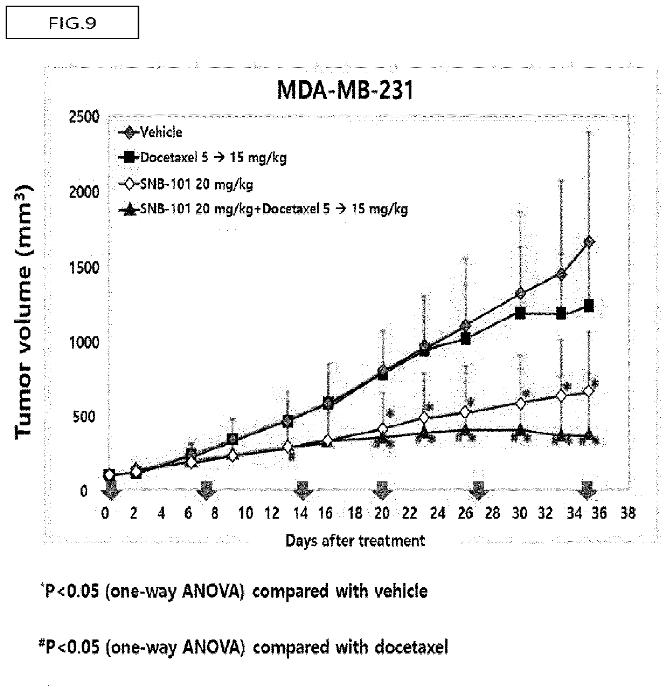

The average tumor volumes of the G1 (Vehicle), G2 (docetaxel), G3 (SNB-101), and G4 (SNB-101 + docetaxel) groups were 1,662.48 mm3, 1,228.93 mm3, 657.30 mm3, and 364.10 mm3, respectively. Co-administration (G4) was more effective in inhibiting tumor formation than single administration (G2, G3). The results are shown in FIG. 9 below.

As of the end of the experiment, relative tumor growth inhibition (TGI) to the vehicle was calculated using the following equation.

Equation 2

Tumor growth inhibition (TGI) (%) = {1-($\triangle$T/$\triangle$C)} x 100

The TGI for each group is summarized in Table 11.

TABLE 11

| Group | TGI (%) |
|---|---|
| G2 (Docetaxel) | 27.6 |
| G3 (SNB-101) | 64.0 |
| G4 (SNB-101 + docetaxel) | 82.6 |

As shown in Table 11, relative TGI to the vehicle was 27.6% in the docetaxel alone group (G2), 64.0% in the SNB-101 alone group (G3), and 82.6% in the SNB-101 + docetaxel combination group (G4). From the above results, it was understood that a synergistic effect appeared upon the co-administration of SNB-101 and docetaxel of the present disclosure to breast cancer cells.

2) Weight measurement result

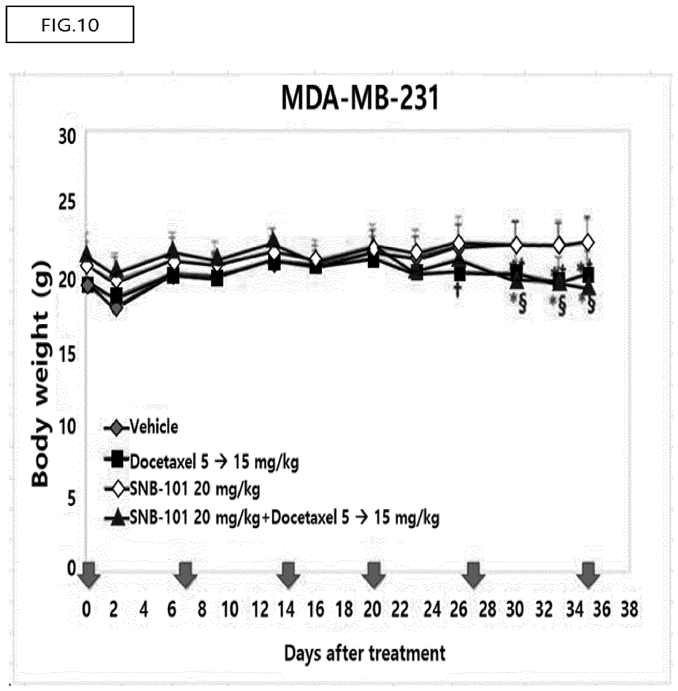

The weight measurement results for each group are depicted in FIG. 10 below.

Body weights in the vehicle, docetaxel, SNB-101, and SNB-101 + docetaxel groups were 19.70 g, 19.63 g, 20.99 g, and 21.46 g, respectively, at the start of drug administration (day 0) and 22.46 g, 20.25 g, 22.43 g, and 19.37 g, respectively, at the end of the test (day 35). That is, the weight loss rate did not exceed 20%, indicating that the mice did not reach the critical minimum weight.

3) Conclusion

Taken together, the data imply that the SNB-101 + docetaxel combination (G4) has an excellent inhibitory effect on tumor growth, compared to docetaxel (G2) and SNB-101 alone (G3), in the breast cancer cell line MDA-MB-231 xenograft models.

4-4. Lung cancer A549 xenograft model test result

1) Tumor volume measurement and result analysis

Compared to G1 (vehicle), tumor growth inhibitory efficacy of G2 (docetaxel alone) or G3 (SNB-101 alone) and G4 (SNB-101 + docetaxel combination) groups were measured on the end of the experiment (37 days after drug administration).

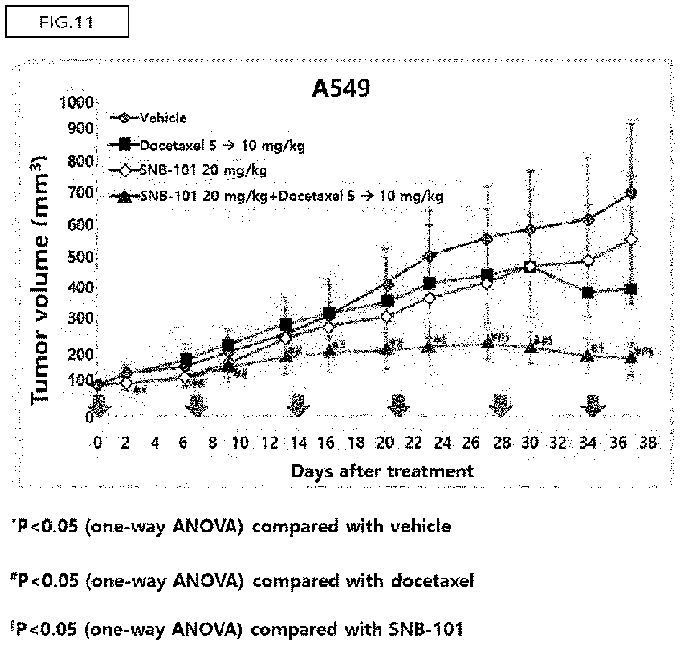

The average tumor volumes of the G1 (Vehicle), G2 (docetaxel), G3 (SNB-101), and G4 (SNB-101 + docetaxel) groups were 704.10 mm3, 402.70 mm3, 557.60 mm3, and 181.08 mm3, respectively. Co-administration (G4) was more effective in inhibiting tumor formation than single administration (G2, G3). The results are shown in FIG.11, below.

As of the end of the experiment, relative tumor growth inhibition (TGI) to the vehicle was calculated using the following equation.

Equation 2

Tumor growth inhibition (TGI)(%) = {1-(△T/△C)} x 100

The TGI for each group is summarized in Table 12.

TABLE 12

| Group | TGI (%) |
|---|---|
| G2 (Docetaxel) | 50.0 |
| G3 (SNB-101) | 24.3 |
| G4 (SNB-101 + docetaxel) | 86.7 |

As shown in Table 12, relative TGI to the vehicle was 50.0% in the docetaxel alone group (G2), 24.3% in the SNB-101 alone group (G3), and 86.7% in the SNB-101 + docetaxel combination group (G4).

From the above results, it was understood that a synergistic effect appeared upon the co-administration of SNB-101 and docetaxel of the present disclosure to the lung cancer cell line A549-xenografted models.

2) Weight measurement result

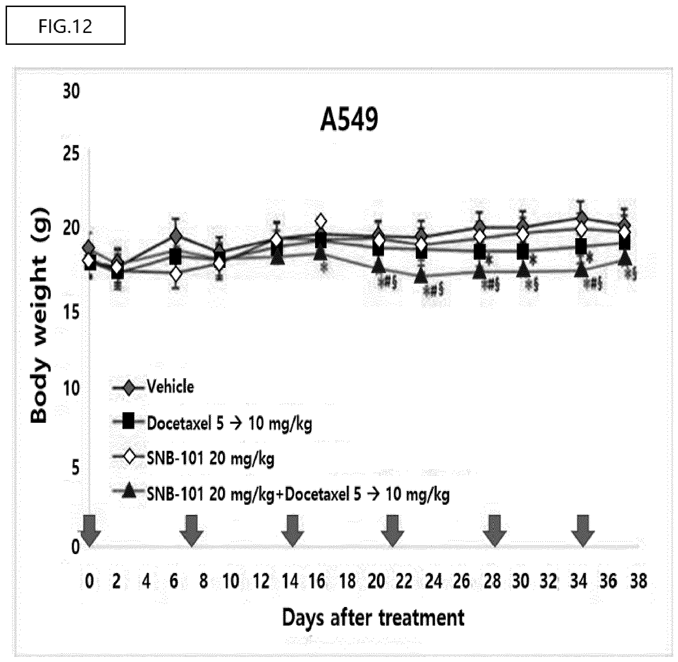

The weight measurement results for each group are depicted in FIG. 12, below.

Body weights in the vehicle, docetaxel, SNB-101, and SNB-101 + docetaxel groups were 17.86 g, 17.93 g, 18.04 g, and 18.68 g, respectively, at the start of drug administration (day 0) and 20.19 g, 19.10 g, 19.79 g, and 17.97 g, respectively, at the end of the test (day 37). That is, the weight loss rate did not exceed 20%, indicating that the mice did not reach the critical minimum weight.

3) Conclusion

Taken together, the data imply that the SNB-101 + docetaxel combination (G4) has an excellent inhibitory effect on tumor growth, compared to docetaxel (G2) and SNB-101 alone (G3), in the lung cancer cell line A549 xenograft models.

EXAMPLE 5: Synergistic Effect of Co-Administration of Nanoparticles (SNB-101) and Bevacizumab (Trade Name: Avastin) in Colorectal Cancer Treatment

An experiment was performed to evaluate the therapeutic synergistic effect of a combination with the bevacizumab, which is a monoclonal antibody and a target anticancer drug, on colorectal cancer.

5-1. Test method

1) Experimental animal

A total of 150 BALB/c nude mice (5-week-old males) were obtained and acclimatized for about one week before transplantation of HT-29 colorectal cancer cells into the subcutaneous area of the right hip. Seven days after transplantation, 50 mice were selected on the basis of the tumor volume and classified into five groups of 10.

2) Tumor cell transplantation

HT-29 (KCLB No. 300038, Korea Cell Line Bank) human colorectal cancer cells were maintained by passage in RPMI 1640 medium supplemented with 10% fetal bovine serum (FBS) and 100 IU/ml penicillin/streptomycin at 37°C in a 5% $CO_2$ incubator. A tumor cell suspension with a density of 3.0x106 cells/0.1ml was transplanted at a dose of 0.1 mL into the subcutaneous area of the right hip of the mice to form a solid tumor mass.

3) Grouping

Eight days after transplantation of the HT-29 colorectal cancer cells, mice were selected based on the tumor volume and randomly divided into 5 groups (10 mice/group). The drug administration group was set as in Table 13, below.

TABLE 13

| Gro up | Drug | Coun t | Dose (mg/kg) | Route |
|---|---|---|---|---|
| G1 | Vehicle (Dextrose 5%) | 10 | - | Venous |
| G2 | SNB-101 | 10 | 20/31.8* | Venous |
| G3 | Bevacizumab | 10 | 5 | Peritoneal |
| G4 | Irinotecan+ Bevacizumab | 10 | 50+5 | Venous + Peritoneal |
| G5 | SNB-101+ Bevacizumab | 10 | 20/31.8*+5 | Venous + Peritoneal |
| *: dose as SN-38/irinotecan HCl | | | | |

4) Drug administration

The test substances SNB-101 and bevacizumab (5 mg/kg) were administered alone or in combination, a total of three times on the same day (days 1, 4, and 7). SNB-101 was administered intravenously at doses calculated on the basis of body weight for individuals while bevacizumab was administered intraperitoneally. An autopsy was performed on day 28. A 5% dextrose solution used as a vehicle for the control was equally administered at a dose of 10 mL/kg.

5-2. Test result

Test results are expressed as mean±S.D. in Table 14, below.

TABLE 14

| Group | Tumor volume (mm3) | | Tumor Volume Change (mm3) [B-A] |
|---|---|---|---|
| | Test start A (day 1) | Test end B (day 28) | |
| G1 | 99.53±10.93 | 1343.90±386.02 | 1244.37±382.42 |
| G2 | 96.32±12.22 | 438.23±168.93 | 341.91±165.88 |
| G3 | 96.67±14.79 | 709.38±266.87 | 612.71±259.64 |
| G4 | 93.27±12.86 | 432.26±91.46 | 338.98±91.09 |
| G5 | 94.88±15.17 | 290.67±114.28 | 195.79±105.71 |

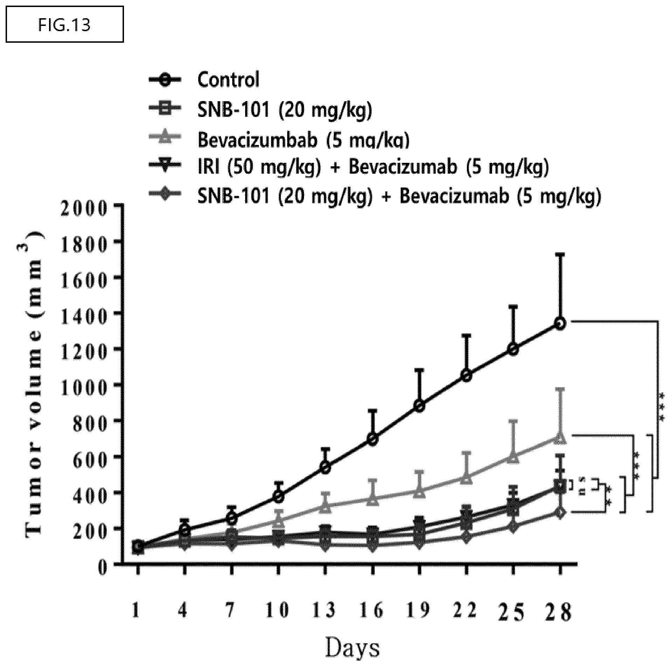

As shown in Table 14, an excellent synergistic therapeutic effect on colorectal cancer was detected from a combination of SNB-101 and bevacizumab (G5), compared to their individual groups SNB-101 (G2) and bevacizumab (G3). A tumor growth plot corresponding to the data in Table 14 is depicted in FIG. 13. Statistical significance for differences in tumor volume at different times between groups was analyzed by two-way ANOVA with Holm-Sidak multiple comparison test (*, $P < 0.05$; **, $P < 0.01$; ***, $P < 0.001$, ns: non-significant).

EXAMPLE 6: Synergistic Effect of Co-Administration of Nanoparticles (SNB-101), Nab-paclitaxel, and Gemcitabine in Pancreatic Cancer Treatment

6-1. Experimental material

- BALB/c-nu mice (Orient Bio, Gapyoung Center), Male, 6 weeks old
- Pancreatic cancer cell line AsPC-1 (ATCC, Cat. No. CRL-1682)
- Test drug: SNB-101 (SN-38 10 mg, irinotecan HCl·3H2O 15.9 mg/vial), nab-paclitaxel (Gelgene Korea, Abraxane, Lot. No. 6200676A), gemcitabine (Korea Lilly, Lot. No. 186053A)
- Vehicle: 5% glucose injection 50 ml (CJ Healthcare, insurance code 640001361)
- RPMI1640 medium (Gibco, A10491-01) supplemented with 10% fetal bovine serum (Gibco, Cat. No. 16000)
- 1% Penicillin-Streptomycin (Gibco, Cat. No. 15140122)
- Trypsin-EDTA (Gibco, Cat. No. 25200-072)
- Cold-DPBS (Hyclone, Cat. No. SH30258.02)
- Trypan blue (Gibco, Cat. No. 15250061)
- MycoAlert Mycoplasma detection kit (LONZA, Cat. No. LT-07-318)
- 150 mm cell culture dish, Conical tubes, Pipettes
- Mouse ear tag, syringe, caliper

6-2. Test method

Pancreatic cancer cell line AsPC-1, supplied by the T2B Infrastructure Center for Digestive Disorders (NCEED), was cultured at 37°C in RPMI1640 medium-10% FBS-1% Penicillin-Streptomycin under a 5% CO2 atmosphere. Thawed cells were cultured for one week or longer, with passages every 2-3 days. The cells were assayed for viability (trypan blue) and infection with bacteria and yeasts (cell images) and mycoplasma (MycoAlert Mycoplasma detection kit). Used for transplantation were the cells that were not infected and exhibited a viability of 90% or higher as measured by trypan blue staining. Cells were floated using trypsin-EDTA and suspended in cold PBS. AsPC-1 cells were subcutaneously implanted at a dose of 5x106 cells/200 μl into the right thigh of BALB/c-nu mice. The mice were periodically monitored for tumor formation and growth, and the tumor volume was calculated using tumor length measurements according to the following equation.

```
[Tumor volume = (a2b)/2, a short diameter, b long
diameter]
```

When the mean tumor volume of the entire mice on the day of administration was 123.6 mm3, they were grouped by paired-matching method. The mice in each group were observed for 2 weeks after administration of the drug according to the dosage regimen shown in Table 15 below.

TABLE 15

| Grou p | Drug | Coun t | Dose (mg/ kg) | Rou te | Frequency and duration |
|---|---|---|---|---|---|
| G1 | Vehicle | 8 | - | i.v | 2QW x 2wk |
| G2 | SNB-101 | 8 | 30/47.7* | i.v | 2QW x 2wk |
| G3 | Nab-paclitaxel + Gemcitabine | 8 | 5+25 | i.v . | 2QW x 2wk |
| G4 | SNB-101+ Nab-paclitaxel + Gemcitabine | 8 | 30/47.7* + 5+25 | i.v . | 2QW x 2wk |
| *: dose as SN-38/irinotecan HCl | | | | | |

Tumor volume and weight were measured twice a week (intervals of 3 or 4 days) from the time of grouping to the end of the test, and the degree of tumor growth inhibition from the start of drug administration to the end of the test was evaluated

6-3. Test result

As of the end of the experiment, relative tumor growth inhibition (TGI) to the vehicle was calculated using Equation 2.

TGI for each group is shown in Table 16.

TABLE 16

| Group | Drug | Count | Dose (mg/kg) | TGI (%) |
|---|---|---|---|---|
| G2 | SNB-101 | 8 | 30/47.7* | 72.5 |
| G3 | Nab-paclitaxel + Gemcitabine | 8 | 5+25 | 56.3 |
| G4 | SNB-101+ Nab-paclitaxel + Gemcitabine | 8 | 30/47.7* +5+25 | 83.4 |

As shown in Table 16, relative TGI to the vehicle was 72.5% in the SNB-101 alone group (G2) and 56.3% in the nab-paclitaxel + gemcitabine group (G3). In contrast, the relative TGI was 83.4% in the SNB-101+nappaclitaxel+gemcitabine combination group (G4), indicating that the co-administration of SNB-101+nab-paclitaxel+gemcitabine is superb in synergistic effect on the treatment of pancreatic cancer.

In addition, apparent side effects, weight loss, serological toxicity were observed in none of the groups.

EXAMPLE 7: Synergistic Effect of Combination of Nanoparticles (SNB-101) and Radiation in Colorectal Cancer and Lung Cancer Treatment

7-1. Experimental material

- BALB/c-nu mice (JoongA Bio), Male, 6 weeks old
- Small cell lung cancer NCI-H69 cell line (ATCC, Cat. No. HTB-119)
- Non-small cell lung cancer A549 cell line (ATCC, Cat. No. CCL-185)
- Colorectal cancer HCT116 cell line (ATCC, Cat. No. CCL-247)
- Test drug: SNB-101 (SN Bioscience Inc., SN-38 10 mg, irinotecan HCl·3H2O 15.9 mg/vial)
- Vehicle: 5% glucose injection 50 ml (CJ Healthcare, Insurance code 640001361)
- RPMI1640 medium (Gibco, 22400-089), F12K (Gibco, 21127-022), McCoy's 5A (Gibco, 16600-082) supplemented with 10% fetal bovine serum (Gibco, Cat. No. 16000)
- 1% antibiotic-antimycotic (Gibco, Cat. No. 15240-062)
- Trypsin-EDTA (Gibco, Cat. No. 25200-072)
- Cold-DPBS (Gibco, Cat. No. 14200-075)
- Trypan blue (Gibco, Cat. No. 15250061)
- MycoAlert Mycoplasma detection kit (LONZA, Cat. No. LT-07-318)
- 150 mm cell culture dish, Conical tubes, Pipettes
- Mouse ear tag, syringe, calipers

7-2. Test method

The frozen cell lines, NCI-H69 (small cell lung cancer), HCT116 (colorectal cancer), and A549 (non-small cell lung cancer), supplied by the T2B Infrastructure Center for Digestive Disorders (NCEED), were thawed and each cultured at 37°C in RPMI1640 medium-10% FBS-1% Penicillin-Streptomycin under a 5% CO2 atmosphere. The thawed cells were cultured for one week or longer, with passages every 2-3 days. The cells were assayed for viability and infection with bacteria and yeasts and mycoplasma (MycoAlert Mycoplasma detection kit). Used for transplantation were the cells that were not infected and exhibited a viability of 90% or higher as measured by trypan blue staining. Cells were floated using trypsin-EDTA and suspended in cold PBS. Subcutaneous cell transplantation was made of NCI-H69 cell at a dose of 1x107 cells/100 μl and A549 cells and HCT116 cells each at a dose of 1x106 cells/50 μl into the right thigh of BALB/c-nu mice. The mice were periodically monitored for tumor formation and growth, and the tumor volume was calculated using tumor length measurements according to the following equation.

[Tumor volume=(a2b)/2, a short diameter, b long diameter]

When the mean tumor volume of the entire mice on the day of administration was $100\pm20$ mm3, they were grouped by paired-matching method. The mice in each group were observed for 2-3 weeks after administration of the drug according to the dosage regimen shown in Tables 17 and 18, below.
NCI-H69 and HCT116 were each injected once a week for one week while A549 was administered five times at a frequency of twice injection a week. After drug administration, measurement was made of tumor volume and body weight in the HCT116 model until 21 days, in the NCI-H69 model until 38 days, and in the A549 model until 45 days.

[common to NCI-H69 (small cell lung cancer), HCT116 (colorectal cancer) xenograft model]

TABLE17

| Group | Drug | Count | Dose (mg/kg) | Route | Frequency and duration |
|---|---|---|---|---|---|
| G1 | Vehicle | 5 | - | i.v. | Single |
| G2 | SNB-101 | 5 | 20/31.8* mg/kg | i.v. | Single |
| G3 | RT | 5 | 5 Gy | External | Single |
| G4 | SNB-101+RT | 5 | 20/31.8* mg/kg + 5 Gy | i.v. External | Single (SNB-101, 2hr before RT) |
| *: dose as SN-38/irinotecan HCl | | | | | |

[common to A549 (non-small cell lung cancer) xenograft model]

TABLE 18

| Group | Drug | Cou nt | Dose (mg/kg) | Route | Frequency and duration |
|---|---|---|---|---|---|
| G1 | Vehicle | 5 | - | i.v. | Single+(9D interval)+BIW X 2*** |
| G2 | SNB-101 | 5 | 20/31.8* mg/k g** | i.v. | Single+(9D interval)+BIW X 2*** |
| G3 | RT | 5 | 5 Gy | Extern al | Single+(14D interval)+Single |
| G4 | SNB-101+RT | 5 | 20/31.8* mg/k g** + 5 Gy | i.vExt ernal | Single+(9D interval)+BIW X 2***Single+ (14D interval)+Single(SNB-101,2hr before RT) |
| *: dose as SN-38/irinotecan HCl<br>**: the dose of SNB-101 administered to the A549 xenograft model was changed in consultation as follows: 20 mg/kg (1st) $\rightarrow$ 30 mg/kg (2nd, death happened) $\rightarrow$ 20 mg/kg (3rd -5th).<br>***: Nine days after the first administration thereof, SNB-101 was administered four times at BIW for 2 weeks (a total of 5 times), and the change in administration frequency was conducted with mutual consensus. | | | | | |

Tumor volume and body weight were measured twice a week (intervals of 3 or 4 days) from the time of grouping to the end of the test, and a tumor growth curve was constructed from the start of drug administration to the end of the test. When the tumor volume reached the limit of the Animal Experimental Ethics Committee (IACUC), the test was terminated by euthanasia in consultation with the sponsor, and the tumor was excised and analyzed.

7-3. Test result

As of the end of the experiment, relative tumor growth inhibition (TGI) to the vehicle was calculated using Equation 2.

For NCI-H69, TGI in each group is shown in Table 19.

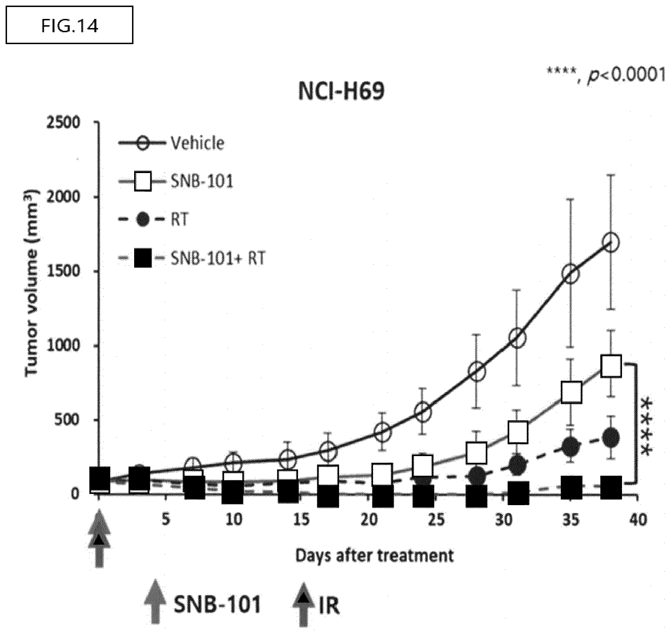

Tumor volume changes are plotted in FIG. 14.

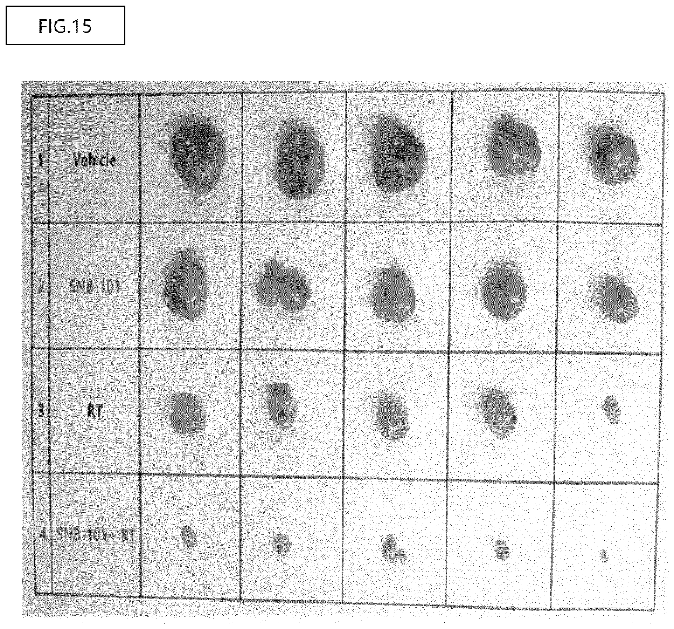

Images of the tumors excised are given in FIG. 15.

TABLE 19

| Grou p | Drug | Cou nt | Dose | TGI (%) |
|---|---|---|---|---|
| G2 | SNB-101 | 5 | 20/31.8 mg/kg | 50.9 |
| G3 | RT | 5 | 5 Gy | 81.3 |
| G4 | SNB-101+RT | 5 | 20/31.8 mg/kg + 5 Gy | 101.5 |

For HCT116, TGI in each group is shown in Table 20.

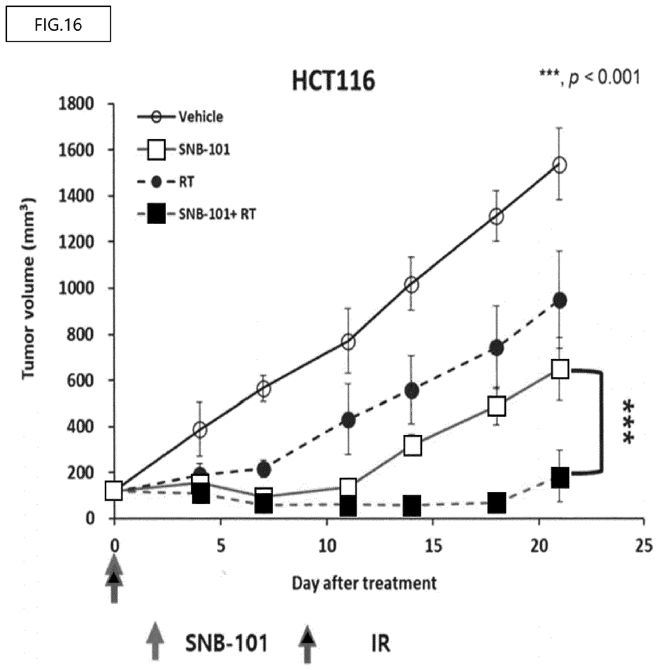

Tumor volume changes are plotted in FIG. 16.

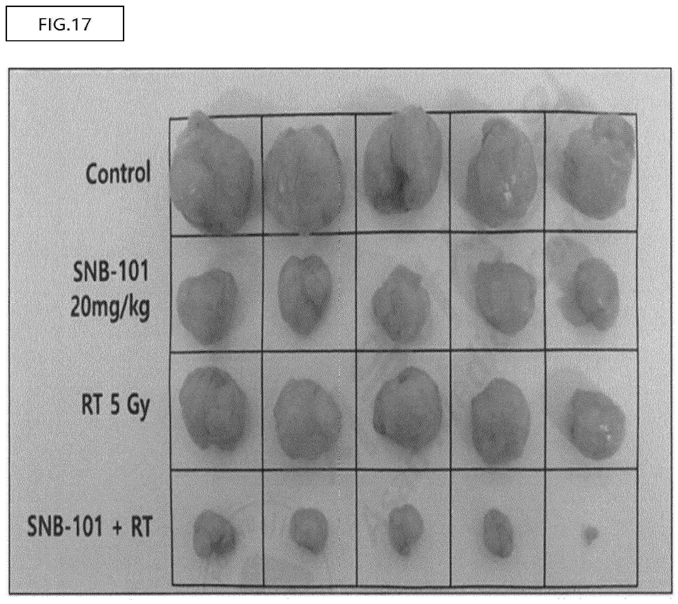

Images of the tumors excised are given in FIG. 17.

TABLE 20

| Group | Drug | Count | Dose | TGI (%) |
|---|---|---|---|---|
| G2 | SNB-101 | 5 | 20/31.8 mg/kg | 62.6 |
| G3 | RT | 5 | 5 Gy | 41.4 |
| G4 | SNB-101+RT | 5 | 20/31. 8 mg/kg + 5 Gy | 95.4 |

For A549, TGI in each group is shown in Table 21.

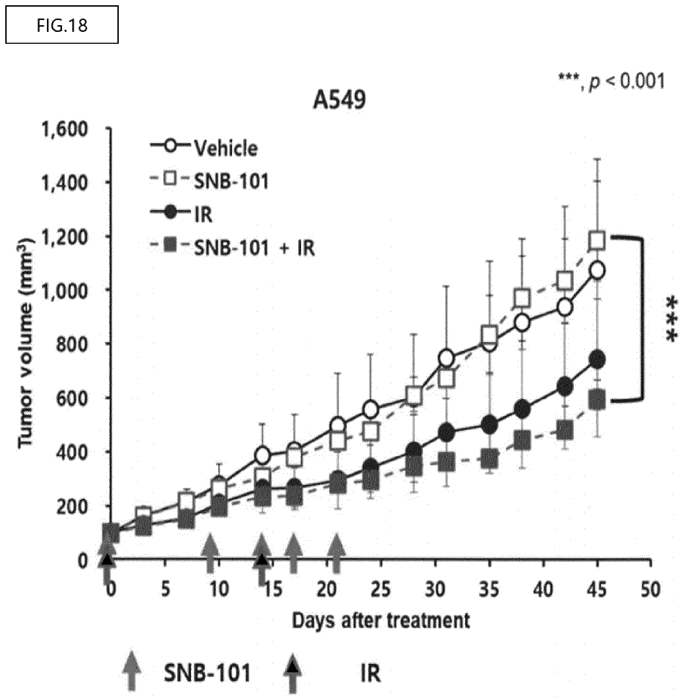

Tumor volume changes are plotted in FIG. 18.

TABLE 21

| Group | Drug | Count | Dose | TGI (%) |
|---|---|---|---|---|
| G2 | SNB-101 | 5 | 20/31.8 mg/kg | -11.2 |
| G3 | RT | 5 | 5 Gy | 33.9 |
| G4 | SNB-101+RT | 5 | 20/31.8 mg/kg + 5 Gy | 49.0 |

As shown in Table 19-21, relative TGI to the vehicle was remarkably higher in the combined treatment group of SNB-101 and radiation (G4) than in the SNB-101 alone group (G2) and the radiation alone (G3). From the data, it was understood that SNB-101 and radiation combined treatment exerted very good synergistical effect on the treatment of colon cancer and lung cancer.

In addition, apparent side effects, weight loss, serological toxicity were observed in none of the groups.

Taken together, the data obtained in Examples 2 to 7 indicate that significant synergistic effects can be achieved in the treatment of cancers when the nanoparticles of the present disclosure are combined with other agents or radiation therapy as summarized in Table 22, below.

TABLE 22

| Ex. | Combination | | Cancer |
|---|---|---|---|
| 2 | SNB-101 | Nab-paclitaxel | Pancreatic cancer |
| 3 | | Docetaxel | Gastric cancer |
| 4 | | Docetaxel | Breast cancer, lung cancer |
| 5 | | Bevacizumab | Colorectal cancer |
| 6 | | Nab-paclitaxel +Gemcitabine | Pancreatic cancer |
| 7 | | Radiation | Colorectal cancer, lung cancer |

Claims

1. A pharmaceutical combination for treating cancer,comprising, as active ingredient,: particles comprising a hydrophobic camptothecin-based compound, a hydrophilic camptothecin-based compound, and an amphiphilic block copolymer composed of a hydrophobic block and a hydrophilic block; and at least one antitumor agent selected from the group consisting of a taxane-based anticancer agent, an albumin-bound taxane-based anticancer agent, a vascular endothelial growth factor (VEGF) inhibitor, and gemcitabine.

2. The pharmaceutical combination of claim 1, wherein the hydrophobic camptothecin-based compound is at least one selected from the group consisting of SN-38 (7-ethyl-10-hydroxy camptothecin), camptothecin, 10-hydroxycamptothecin, and pharmaceutically acceptable salts thereof.

3. The pharmaceutical combination of claim 1, wherein the hydrophilic camptothecin-based compound is at least one selected from the group consisting of irinotecan, topotecan, belotecan, exatecan, lurtotecan, sinotecan, rubitecan, 9-nitrocamptothecin, 9-aminocamptothecin, gimatecan, karenitecin, silatecan, diflomotecan, elomotecan, a pharmaceutically acceptable salt thereof, a glucuronide metabolite thereof, and a glucuronide metabolite of the hydrophobic camptothecin-based compound.

4. The pharmaceutical combination of claim 1, wherein the amphiphilic block copolymer is composed of A-B blocks or A-B-A blocks, wherein (a) A is a hydrophilic polymer, which is monomethoxy polyethylene glycol, dimethoxy polyethylene glycol, polyethylene glycol, polypropylene glycol, monomethoxy polypropylene glycol, polyethylene oxide, polyacrylic acid, or a polymer thereof; and (b) B is a hydrophobic polymer, which is polylactic acid, poly-L-lactide, poly-D-lactide, poly-D, L-lactide, poly(lactide-co-glycolide), polyglycolic acid, polyglycolide, a polylactic acid-glycolic acid copolymer, polymandelic acid, polycaprolactone, polydioxan-2-one, polyglutamic acid, polyaspartic acid, polyornithine, polyorthoester, or a derivative thereof.

5. The pharmaceutical combination of claim 1, wherein the vascular endothelial growth factor (VEGF) inhibitor is at least one selected from the group consisting of bevacizumab, ranibizumab, and aflibercept.

6. The pharmaceutical combination of any one of claims 1 to 5, wherein the particles have an average diameter of 2 to 200 nm.

7. The pharmaceutical combination of any one of claims 1 to 5, wherein the cancer is selected from the group consisting of gastric cancer, ovarian cancer, uterine cancer, cervical cancer, small cell lung cancer, non-small cell lung cancer, pancreatic cancer, breast cancer, colorectal cancer, esophageal cancer, retinoblastoma, oral cancer, salivary gland cancer, laryngeal cancer, pharyngeal cancer, kidney cancer, prostate cancer, melanoma, liver cancer, gallbladder cancer, biliary tract cancer, thyroid cancer, bladder cancer, brain cancer, central nervous system cancer, bone tumor, skin cancer, non-Hodgkin's lymphoma, and non-Hodgkin's lymphoma.

8. A pharmaceutical composition for treating cancer, wherein the composition comprises particles comprising a hydrophobic camptothecin-based compound, a hydrophilic camptothecin-based compound, and an amphiphilic block copolymer composed of a hydrophobic block and a hydrophilic block, and is used in combination with radiotherapy (RT).

9. A method for treating cancer, the method comprising a step of administering, to a subject in need of administration, (a) a first pharmaceutical composition comprising particles comprising a hydrophobic camptothecin-based compound, a hydrophilic camptothecin-based compound, and an amphiphilic block copolymer composed of a hydrophobic block and a hydrophilic block in combination with (b) antitumor therapy or a second pharmaceutical composition comprising at least one antitumor agent as an active ingredient selected from the group consisting of a taxane-based anticancer agent, an albumin-bound taxane-based anticancer agent, a vascular endothelial growth factor (VEGF) inhibitor, and gemcitabine.

10. The method of claim 9, wherein the first pharmaceutical composition and the second pharmaceutical composition are administered simultaneously, separately, or sequentially.

11. The method of claim 9, wherein the first pharmaceutical composition and the second pharmaceutical composition are administered as a composite formulation or a single formulation.

12. The method of claim 9, wherein the hydrophobic camptothecin-based compound is at least one selected from the group consisting of SN-38 (7-ethyl-10-hydroxy camptothecin), camptothecin, 10-hydroxycamptothecin, and pharmaceutically acceptable salts thereof.

13. The method of claim 9, wherein the hydrophilic camptothecin-based compound is at least one selected from the group consisting of irinotecan, topotecan, belotecan, exatecan, lurtotecan, sinotecan, rubitecan, 9-nitrocamptothecin, 9-aminocamptothecin, gimatecan, karenitecin, silatecan, diflomotecan, elomotecan, a pharmaceutically acceptable salt thereof, a glucuronide metabolite thereof, and a glucuronide metabolite of the hydrophobic camptothecin-based compound.

14. The method of claim 9, wherein the amphiphilic block copolymer is composed of A-B blocks or A-B-A blocks, wherein (a) A is a hydrophilic polymer, which is monomethoxy polyethylene glycol, dimethoxy polyethylene glycol, polyethylene glycol, polypropylene glycol, monomethoxy polypropylene glycol, polyethylene oxide, polyacrylic acid, or a polymer thereof; and (b) B is a hydrophobic polymer, which is polylactic acid, poly-L-lactide, poly-D-lactide, poly-D, L-lactide, poly(lactide-co-glycolide), polyglycolic acid, polyglycolide, a polylactic acid-glycolic acid copolymer, polymandelic acid, polycaprolactone, polydioxan-2-one, polyglutamic acid, polyaspartic acid, polyornithine, polyorthoester, or a derivative thereof.

15. The method of claim 9, wherein the vascular endothelial growth factor (VEGF) inhibitor is at least one selected from the group consisting of bevacizumab, ranibizumab, and aflibercept.

16. The method of claim 9, wherein the antitumor therapy is surgery, radiotherapy, or a combination thereof.

17. The method of claim 9, wherein the particles have an average diameter of 2 to 200 nm.

18. The method of claim 9, wherein the cancer is selected from the group consisting of gastric cancer, ovarian cancer, uterine cancer, cervical cancer, small cell lung cancer, non-small cell lung cancer, pancreatic cancer, breast cancer, colorectal cancer, esophageal cancer, retinoblastoma, oral cancer, salivary gland cancer, laryngeal cancer, pharyngeal cancer, kidney cancer, prostate cancer, melanoma, liver cancer, gallbladder cancer, biliary tract cancer, thyroid cancer, bladder cancer, brain cancer, central nervous system cancer, bone tumor, skin cancer, non-Hodgkin's lymphoma, and non-Hodgkin's lymphoma.

FIG.1

FIG.2

FIG.3

FIG.4

FIG.5

FIG.6

FIG.7

FIG.8

FIG.9

*P<0.05 (one-way ANOVA) compared with vehicle

#P<0.05 (one-way ANOVA) compared with docetaxel

FIG.10

FIG.11

A549

*P<0.05 (one-way ANOVA) compared with vehicle

#P<0.05 (one-way ANOVA) compared with docetaxel

§P<0.05 (one-way ANOVA) compared with SNB-101

FIG.12

A549

Body weight (g)

Days after treatment

Legend:
- ◆ Vehicle
- ■ Docetaxel 5 → 10 mg/kg
- ◇ SNB-101 20 mg/kg
- ▲ SNB-101 20 mg/kg+Docetaxel 5 → 10 mg/kg

FIG.13

Legend:
- Control
- SNB-101 (20 mg/kg)
- Bevacizumbab (5 mg/kg)
- IRI (50 mg/kg) + Bevacizumab (5 mg/kg)
- SNB-101 (20 mg/kg) + Bevacizumab (5 mg/kg)

Y-axis: Tumor volume ($mm^3$)

X-axis: Days

FIG.14

NCI-H69

****, *p*<0.0001

Vehicle
SNB-101
RT
SNB-101+ RT

SNB-101    IR

Days after treatment

FIG.15

FIG.16

FIG.17

FIG.18

# EP 4 252 747 A1

## INTERNATIONAL SEARCH REPORT

| International application No. |
|---|
| **PCT/KR2021/017296** |

**A. CLASSIFICATION OF SUBJECT MATTER**

**A61K 9/51**(2006.01)i; **A61K 31/437**(2006.01)i; **A61K 31/4745**(2006.01)i; **A61K 45/06**(2006.01)i; **A61P 35/00**(2006.01)i

According to International Patent Classification (IPC) or to both national classification and IPC

**B. FIELDS SEARCHED**

Minimum documentation searched (classification system followed by classification symbols)

A61K 9/51(2006.01); A61K 31/4745(2006.01); A61K 9/107(2006.01); A61K 9/50(2006.01); A61P 35/00(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 캄토테신 (camptothecin), 이리노테칸 (ironotecan), 베바시주맙 (bevacizumab), 양친매성 블록 공중합체 (amphiphilic block copolymer), 방사선 (radiation), 폴리에틸렌글리콜 (polyethylene glycol)

**C. DOCUMENTS CONSIDERED TO BE RELEVANT**

| Category* | Citation of document, with indication, where appropriate, of the relevant passages | Relevant to claim No. |
|---|---|---|
| X | US 2018-0369231 A1 (SNBIOSCIENCE INC.) 27 December 2018 (2018-12-27)<br>See claims 1-12 and paragraphs [0033] and [0098]. | 1-4,6,7 |
| Y | | 5,8 |
| Y | KENMOTSU, H. et al. The Antitumor Activity of NK012, an SN-38–Incorporating Micelle, in Combination With Bevacizumab Against Lung Cancer Xenografts. Cancer. 2010, vol. 116, pp. 4597-4604.<br>See abstract and page 4597. | 5 |
| Y | SAIJO, N. Irinotecan Combined with Radiation Therapy for Patients with Stage III Non–Small-Cell Lung Cancer: Current Trials. Clinical Lung Cancer. 2002, vol. 4, Supplement 1, S21-S25.<br>See abstract and page S24, left column. | 8 |
| A | CN 109303780 A (YANTAI INSTITUTE OF MATERIA MEDICA) 05 February 2019 (2019-02-05)<br>See claims 1 and 2. | 1-8 |
| A | EP 3231423 A1 (JENKEM TECHNOLOGY CO., LTD. (TIANJIN)) 18 October 2017 (2017-10-18)<br>See claim 1. | 1-8 |

☐ Further documents are listed in the continuation of Box C.  ☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| | |
|---|---|
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **02 March 2022** | **02 March 2022** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

44

**INTERNATIONAL SEARCH REPORT**

International application No.

**PCT/KR2021/017296**

| Box No. II | Observations where certain claims were found unsearchable (Continuation of item 2 of first sheet) |

This international search report has not been established in respect of certain claims under Article 17(2)(a) for the following reasons:

1. ☑ Claims Nos.: **9-18**
because they relate to subject matter not required to be searched by this Authority, namely:

Claims 9-18 pertain to a method for treatment of the human body by surgery or therapy, as well as a diagnostic method (PCT Article 17(2)(a)(i) and PCT Rule 39.1(iv)).

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2019)

**INTERNATIONAL SEARCH REPORT**
Information on patent family members

International application No.

**PCT/KR2021/017296**

| Patent document cited in search report | | | Publication date (day/month/year) | Patent family member(s) | | | Publication date (day/month/year) |
|---|---|---|---|---|---|---|---|
| US | 2018-0369231 | A1 | 27 December 2018 | AU | 2018-289188 | A1 | 05 December 2019 |
| | | | | AU | 2018-289188 | A1 | 27 December 2018 |
| | | | | AU | 2018-289188 | B2 | 24 December 2020 |
| | | | | BR | 112019024744 | A2 | 16 June 2020 |
| | | | | CA | 3064564 | A1 | 27 December 2018 |
| | | | | CA | 3064564 | C | 30 November 2021 |
| | | | | CN | 110769813 | A | 07 February 2020 |
| | | | | DK | 3641730 | T3 | 03 May 2021 |
| | | | | EP | 3641730 | A1 | 29 April 2020 |
| | | | | EP | 3641730 | B1 | 03 March 2021 |
| | | | | ES | 2865055 | T3 | 14 October 2021 |
| | | | | HR | P20210767 | T1 | 23 July 2021 |
| | | | | HU | E053929 | T2 | 28 July 2021 |
| | | | | IL | 270818 | D0 | 30 January 2020 |
| | | | | JP | 2020-524712 | A | 20 August 2020 |
| | | | | KR | 10-2019-0000325 | A | 02 January 2019 |
| | | | | KR | 10-2094543 | B1 | 27 March 2020 |
| | | | | PL | 3641730 | T3 | 23 August 2021 |
| | | | | PT | 3641730 | T | 01 April 2021 |
| | | | | RU | 2745070 | C1 | 18 March 2021 |
| | | | | SI | 3641730 | T1 | 31 August 2021 |
| | | | | US | 10980796 | B2 | 20 April 2021 |
| | | | | US | 2021-0228568 | A1 | 29 July 2021 |
| | | | | US | 2021-0228569 | A1 | 29 July 2021 |
| | | | | US | 2021-0236480 | A1 | 05 August 2021 |
| | | | | US | 2021-0308122 | A1 | 07 October 2021 |
| | | | | WO | 2018-236190 | A1 | 27 December 2018 |
| CN | 109303780 | A | 05 February 2019 | CN | 109303780 | B | 21 September 2021 |
| EP | 3231423 | A1 | 18 October 2017 | CN | 104524588 | A | 22 April 2015 |
| | | | | CN | 110448533 | A | 15 November 2019 |
| | | | | EP | 3231423 | B1 | 19 August 2020 |
| | | | | US | 10391063 | B2 | 27 August 2019 |
| | | | | US | 2017-0266305 | A1 | 21 September 2017 |
| | | | | WO | 2016-086847 | A1 | 09 June 2016 |

Form PCT/ISA/210 (patent family annex) (July 2019)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 11020200163156

- KR 102094543

**Non-patent literature cited in the description**

- Remington's Pharmaceutical Sciences. 1995